(19) Europäisches Patentamt
European Patent Office
Office européen des brevets

(11) EP 2 545 900 B1

(12) **EUROPÄISCHE PATENTSCHRIFT**

(45) Veröffentlichungstag und Bekanntmachung des Hinweises auf die Patenterteilung:
**08.08.2018 Patentblatt 2018/32**

(21) Anmeldenummer: **12172135.1**

(22) Anmeldetag: **15.06.2012**

(51) Int Cl.:
***A61K 8/34*** (2006.01) ***A61K 8/35*** (2006.01)
***A61K 8/37*** (2006.01) ***A61K 8/40*** (2006.01)
***A61K 8/49*** (2006.01) ***A61K 8/55*** (2006.01)
***A61Q 17/04*** (2006.01) ***A61K 8/06*** (2006.01)

(54) **Sonnenschutzzusammensetzungen mit hohem Lichtschutzfaktor und angenehmer Sensorik**

Sun screen compounds with high sun block factor and pleasant feel

Compositions de protection solaire avec un facteur élevé de protection contre la lumière et capteur adapté

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **15.07.2011 DE 102011079239**

(43) Veröffentlichungstag der Anmeldung:
**16.01.2013 Patentblatt 2013/03**

(73) Patentinhaber: **Henkel AG & Co. KGaA**
**40589 Düsseldorf (DE)**

(72) Erfinder:
- **Weiß, Thomas**
**40591 Düsseldorf (DE)**
- **Dickhof, Susanne**
**41748 Viersen (DE)**

(56) Entgegenhaltungen:
**EP-A2- 2 174 645**
**EP-A2- 2 201 927**
**EP-A2- 2 205 202**
**FR-A1- 2 939 669**
**KR-A- 20080 064 468**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 545 900 B1

## Beschreibung

Die vorliegende Erfindung betrifft Sonnenschutzzusammensetzungen mit hohem Lichtschutzfaktor zum besonders wirksamen Schutz von Haut und Haar gegen UV-Strahlung, die eine besonders angenehme Sensorik aufweisen.

Die hautschädigende Wirkung des ultravioletten Teils der Sonnenstrahlung ist bekannt. Die UV-Strahlung im Bereich zwischen 290 nm und 320 nm, dem so genannten UV-B-Bereich, kann zu einem Erythem, einem einfachen Sonnenbrand oder sogar mehr oder weniger starken Verbrennungen als akuten Erscheinungsformen führen. Als ein Maximum der Erythemwirksamkeit des Sonnenlichtes wird der engere Bereich um 308 nm angegeben.

Man hat lange Zeit fälschlicherweise angenommen, dass die längerwellige UV-A-Strahlung mit einer Wellenlänge zwischen 320 nm und 400 nm nur eine vernachlässigbare biologische Wirkung aufweist. Inzwischen ist allerdings durch zahlreiche Studien belegt, dass UV-A-Strahlung im Hinblick auf die Auslösung photodynamischer, speziell phototoxischer Reaktionen und chronischer Veränderungen der Haut, weitaus gefährlicher als UV-B-Strahlung ist. Auch kann der schädigende Einfluss der UV-B-Strahlung durch UV-A-Strahlung noch verstärkt werden. So ist es u. a. erwiesen, dass selbst die UV-A-Strahlung unter ganz normalen Alltagsbedingungen ausreicht, um innerhalb kurzer Zeit die Collagen- und Elastinfasern zu schädigen, die für die Struktur und Festigkeit der Haut von wesentlicher Bedeutung sind. Hierdurch kommt es zu chronischen lichtbedingten Hautveränderungen - die Haut "altert" vorzeitig. Zum klinischen Erscheinungsbild der durch Licht gealterten Haut gehören beispielsweise Falten und Fältchen sowie ein unregelmäßiges, zerfurchtes Relief. Ferner können die von lichtbedingter Hautalterung betroffenen Partien eine unregelmäßige Pigmentierung aufweisen. Auch die Bildung von braunen Flecken, Keratosen und sogar Karzinomen bzw. malignen Melanomen ist möglich. Eine durch die alltägliche UV-Belastung vorzeitig gealterte Haut zeichnet sich außerdem durch eine geringere Aktivität der Langerhans-Zellen und eine leichte, chronische Entzündung aus.

Etwa 90 % der auf die Erde gelangenden ultravioletten Strahlung besteht aus UV-A-Strahlen. Während die UV-B-Strahlung in Abhängigkeit von zahlreichen Faktoren stark variiert (z. B. Jahres- und Tageszeit oder Breitengrad), bleibt die UV-A-Strahlung unabhängig von jahres- und tageszeitlichen oder geographischen Faktoren Tag für Tag relativ konstant. Gleichzeitig dringt der überwiegende Teil der UV-A-Strahlung in die lebende Epidermis ein, während etwa 70 % der UV-B-Strahlen von der Hornschicht zurückgehalten werden.

Es ist daher von grundsätzlicher Wichtigkeit, dass kosmetische und dermatologische Lichtschutzzusammensetzungen sowohl gegen UV-B- als auch gegen UV-A-Strahlung ausreichenden Schutz bieten. Hierzu sind im Stand der Technik schon eine Vielzahl von kosmetischen Zusammensetzungen entwickelt worden, die mindestens eine UV-Filtersubstanz enthalten.

Bei den UV-Filtersubstanzen handelt es sich um bei Raumtemperatur flüssig oder kristallin vorliegende Substanzen, die in der Lage sind, ultraviolette Strahlen zu absorbieren und die aufgenommene Energie in Form längerwelliger Strahlung, z. B. Wärme, wieder abzugeben. Man unterscheidet UV-A-Filter und UV-B-Filter, je nachdem, in welchem Wellenlängenbereich das Absorptionsmaximum des Filters liegt.

Organische UV-Filter in den für einen hohen Lichtschutzfaktor notwendigen Mengen lassen sich häufig nur mit großen Schwierigkeiten so in kosmetische Träger einarbeiten, dass langzeitstabile Zusammensetzungen mit angenehmen, insbesondere nicht-klebrigen Eigenschaften, erhalten werden.

Zur Herstellung besonders leistungsstarker Lichtschutzzusammensetzungen in Form von Öl-Wasser-Emulsionen (Öl-in-Wasser oder Wasser-in-Öl) ist es vorteilhaft, wenn sowohl in der Ölphase als auch in der Wasserphase mindestens ein organischer UV-Filter enthalten ist. Da UV-Filter in großen Wirtschaftsräumen, wie den USA, der EU, Japan oder Australien, zulassungspflichtige Substanzen darstellen, ist der Kosmetikfachmann bei der Herstellung neuer Lichtschutzzusammensetzungen an eine relativ begrenzte Palette zugelassener UV-Filter gebunden. Ein besonders bevorzugter wasserlöslicher organischer UV-Filter hieraus ist 2-Phenylbenzimidazol-5-sulfonsäure. Diese Verbindung neigt allerdings dazu, bei längerer Lagerung auszukristallieren, was den Lichtschutzfaktor (Sun Protection Factor, SPF) der gesamten Zusammensetzung drastisch absinken lässt.

EP 2201927 A2 beschäftigt sich mit ähnlichen Aufgabenstellungen wie die vorliegende Anmeldung und löst diese mit Sonnenschutzzusammensetzungen in Form von Öl-in-Wasser-Emulsionen, enthaltend mindestens einen öllöslichen und/oder wasserlöslichen organischen UV-Filter, mindestens einen anionischen Öl-in-Wasser-Emulgator, ausgewählt aus den Salzen von $C_{12-20}$-Alkylphosphat, insbesondere aus den Salzen von Cetylphosphat, und mindestens ein $C_{14-20}$-Mono- oder Diacylglycerid, bevorzugt mindestens ein $C_{16-18}$-Mono- oder Diacylglycerid, besonders bevorzugt ausgewählt aus gehärteten Palmölglyceriden, wobei die Zusammensetzungen einen pH-Wert bei 20°C von 6,0 - 8,0 aufweisen. Bevorzugte Zusammensetzungen enthalten die UV-Filterkombination aus 4-(tert.-Butyl)-4'-methoxydibenzoylmethan, Polysilicone-15, 2-Cyano-3,3-phenylzimtsäure-2-ethylhexyl-ester und mindestens einem wasserlöslichen UV-Filter, ausgewählt aus mindestens einem Derivat der Benzimidazolsulfonsäure. Der UV-Filter Polysilicone-15 ist in allen Formulierungsbeispielen enthalten und trägt wesentlich zu einem hohen SPF bei. Es zeigte sich allerdings, dass diese Formulierungen bei der Anwendung auf der Haut stark migrierten und so Irritationen, insbesondere an den Augen, verursachten, was bei den Benutzern zur Ablehnung der Produkte führte. Ohne an diese Theorie gebunden sein zu wollen, wird vermutet, dass die Augenunverträglichkeit auf den Gehalt an Polysilicone-15 zurückzuführen war.

Aufgaben

Eine Aufgabe der vorliegenden Erfindung war es, Lichtschutzzusammensetzungen bereitzustellen, die einen hohen Schutz gegen die oben genannten verschiedenen nachteiligen Wirkungen der Strahlung im UV-A-und UV-B-Bereich, insbesondere Sonnenstrahlung, bieten und gleichzeitig eine hohe Augen- und Schleimhautverträglichkeit aufweisen.

Eine Aufgabe der vorliegenden Erfindung war es, Lichtschutzzusammensetzungen bereitzustellen, die einen hohen Schutz gegen die oben genannten verschiedenen nachteiligen Wirkungen der Strahlung im UV-A-und UV-B-Bereich, insbesondere Sonnenstrahlung, bieten und gleichzeitig eine hohe Lagerstabilität aufweisen.

Weiterhin sollten die erfindungsgemäßen Zusammensetzungen mit einer möglichst geringen Konzentration an möglichst gut Haut-, Augen- und Schleimhautverträglichen organischen UV-Filtern einen möglichst hohen Lichtschutzfaktor (SPF) erzielen. Besonders bevorzugt sollte ein SPF von mindestens 30, außerordentlich bevorzugt von mindestens 40, erzielt werden. Weiterhin sollte versucht werden, die Rezepturbasis so zu optimieren, dass für eine bestimmte UV-Filtermenge und -kombination ein möglichst hoher Lichtschutzfaktor (SPF) erzielt werden kann ("SPF boosting"). Eine weitere Aufgabe der vorliegenden Erfindung war es, Lichtschutzzusammensetzungen bereitzustellen, die neben einem hohen Lichtschutzfaktor (SPF) eine gute Sensorik aufweisen, das heißt, die sich mit angenehmer Haptik entnehmen und auftragen lassen und bei und nach der Applikation ein angenehmes Hautgefühl erzielen.

Im September 2006 wurde eine Empfehlung der EU-Kommission (EU Commission Recommendation of 22 September 2006 on the efficacy of sunscreen products and the claims made relating thereto (2006/647/EC)) herausgegeben, nach der Sonnenschutzmittel ein Mindestmaß an UV-A-Schutz bieten sollen. Sofern die Schutzleistung (UV-A-Schutzfaktor) gegenüber dem Lichtschutzfaktor größer als ein Drittel ist, kann das Produkt ein neues Logo für ausreichenden UV-A-Schutz tragen (siehe EU-Kommissions-Empfehlung 2006/647/EC, Abschnitt 3, Absatz 10b). Zur Bestimmung des UV-A-Schutzfaktors erlaubt die EU Empfehlung eine in vivo-Methode oder in vitro-Methoden, für die belegt ist, dass sie mit in vivo-Methoden korrelieren.

Dementsprechend war es eine weitere bevorzugte Unteraufgabe der vorliegenden Erfindung, eine Sonnenschutzzusammensetzung mit hoher Augen- und Schleimhautverträglichkeit bereitzustellen, die die Anforderung der EU-Kommissions-Empfehlung 2006/647/EC, Abschnitt 3, Absatz 10b, erfüllt und ein Verhältnis von Lichtschutzfaktor zu UV-A-Schutzfaktor unter 3 aufweist:

$$\frac{\text{SPF}_{\text{in vivo}}}{\text{UVAPF}} < 3$$

$$\text{UVAPF} = \frac{\int_{\lambda=320nm}^{\lambda=400nm} P(\lambda) * I(\lambda) * d\lambda}{\int_{\lambda=320nm}^{\lambda=400nm} P(\lambda) * I(\lambda) * 10^{-A(\lambda)*C} * d\lambda}$$

mit

P(λ) = PPD-Wirkungsspektrum (PPD = Persistent Pigment Darkening, siehe V. Wendel et al., The influence of pre-irradiation on the predictability of an in vivo UVA protection with a new in vitro method, Photoder matol. Photoimmunol. Photomed., Band 19, Seiten 93-97)

I(λ) = Beleuchtungsstärke (spectral irradiance) der UV-Quelle (UVA 320-400 nm für PPD-Test)

C = Korrekturfaktor; iterativ bestimmt, um den in vitro SPF an den in vivo SPF anzupassen (siehe unten); sollte einen Wert von 0,8 bis 1,2 haben

d(λ) = 1 nm

A(λ) = mittlere monochromatische Absorption des Testprodukts nach UV-Bestrahlung

$$SPF_{in\ vitro} = \frac{\int_{\lambda=290nm}^{\lambda=400nm} E(\lambda) * I(\lambda) * d\lambda}{\int_{\lambda=290nm}^{\lambda=400nm} E(\lambda) * I(\lambda) * 10^{-A_0(\lambda)} * d\lambda}$$

mit

| | |
|---|---|
| $E(\lambda)$ | = Erythem-Wirkungsspektrum (CIE - 1987) |
| $I(\lambda)$ | = Beleuchtungsstärke (spectral irradiance) der UV-Quelle (Sonnenlichtspektrum für SPF-Test) |
| $A_0(\lambda)$ | = mittlere monochromatische Absorption des Testprodukts vor UV-Bestrahlung |
| $d(\lambda)$ | = 1 nm |

$$SPF_{in\ vitro,adj} = SPF\ labelled = \frac{\int_{\lambda=290nm}^{\lambda=400nm} E(\lambda) * I(\lambda) * d\lambda}{\int_{\lambda=290nm}^{\lambda=400nm} E(\lambda) * I(\lambda) * 10^{-A_0(\lambda) * C} * d\lambda}$$

SPF labelled = SPF in vivo

$$UVAPF_0 = \frac{\int_{\lambda=320nm}^{\lambda=400nm} P(\lambda) * I(\lambda) * d\lambda}{\int_{\lambda=320nm}^{\lambda=400nm} P(\lambda) * I(\lambda) * 10^{-A_0(\lambda) * C} * d\lambda}$$

Die UVA-Strahlendosis D, die für die Messungen nötig ist, um eine angemessene Korrelation zwischen UVAPF in vitro und in vivo PPD-Werten zu erhalten, berechnet sich nach COLIPA-Empfehlung wie folgt: $D = 1{,}2 \cdot UVAPF_0$ J/cm$^2$.
Weiterhin sollten erfindungsgemäße Zusammensetzungen mit einem möglichst langzeit- und lagerstabilen SPF bereitgestellt werden, deren anfänglich gemessener SPF nach mehrwöchiger Lagerung möglichst wenig abnimmt.
Eine weitere Aufgabe der vorliegenden Erfindung war es, Lichtschutzzusammensetzungen, die mindestens einen wasserlöslichen organischen UV-Filter enthalten, in lagerstabiler Form bereitzustellen.
Diese Aufgaben werden in überraschender Weise vom Gegenstand der Ansprüche der vorliegenden Anmeldung gelöst.
Gegenstand der vorliegenden Anmeldung sind kosmetische Sonnenschutz-Zusammensetzungen in Form einer Öl-in-Wasser-Emulsion, enthaltend die Komponenten a) bis g),

a) mindestens ein Salz eines $C_{12-20}$-Alkylphosphats, insbesondere ein Salz von Cetylphosphat, als anionischen Öl-in-Wasser-Emulgator, in einer Gesamtmenge von 0,5 - 2,0 Gew.-%,
b) mindestens ein $C_{14-20}$-Mono- oder Diacylglycerid, bevorzugt mindestens ein $C_{16-18}$-Mono- oder Diacylglycerid, besonders bevorzugt ausgewählt aus gehärteten Palmölglyceriden, in einer Gesamtmenge von 0,3 - 1,7 Gew.-%,
c) 0,3 - 1 Gew.-% Behenylalkohol,
d) lineare primäre Alkanole mit 12 bis 21 oder mehr als 22 Kohlenstoffatomen in einer Gesamtmenge von 0,03 bis 0,4 Gew.-%,
e) in einer Gesamtmenge von 0,7 - 2 Gew.-% n-Hexadecyl-n-nonanoat, n-Octadecyl-n-nonanoat oder eine Mischung hiervon,
f) 2 - 6 Gew.-% 2-Ethylhexyl-3,5,5-trimethylhexanoat,

g) mindestens eine in der Ölphase oder der Wasserphase gelöste organische UV-Filtersubstanz, darunter 2-Cyano-3,3-phenylzimtsäure-2-ethylhexylester, 4-(tert.-Butyl)-4'-methoxydibenzoylmethan und mindestens eine in der Wasserphase gelöste organische UV-Filtersubstanz, die ausgewählt ist aus Phenylen-1,4-bis-(2-benzimidazyl)-3,3'-5,5'-tetrasulfonsäure, 2-Phenylbenzimidazol-5-sulfonsäure und den Salzen dieser Säuren sowie aus Mischungen der genannten Sulfonsäurederivate, in einer Gesamtmenge von mindestens 15 Gew.-%, dadurch gekennzeichnet, dass die mindestens eine in der Ölphase gelöste organische UV- Filtersubstanz 3,3,5-Trimethylcyclohexylsalicylat (Homosalat) umfasst, wobei sich die Angaben in Gew.-% auf das Gesamtgewicht der Sonnenschutz-Zusammensetzung beziehen.

Überraschend wurde festgestellt, dass sich mit den obligatorischen Komponenten a) bis g) Sonnenschutz-Zusammensetzungen mit hohem Lichtschutzfaktor und dennoch angenehmer Sensorik, insbesondere angenehmer Haptik, formulieren lassen.

Erfindungsgemäß bevorzugte Salze von $C_{12-20}$-Alkylphosphaten, die als anionischer Öl-in-Wasser-Emulgator einen ersten Teil des erfindungsgemäß eingesetzten O/W-Emulgatorsystems bilden, sind ausgewählt aus den Monoestern von Phosphorsäure mit Laurylalkohol, Tridecylalkohol, Isotridecylalkohol, Myristylalkohol, Pentadecylalkohol, Cetylalkohol, Palmitylalkohol, Isocetylalkohol, Isostearylalkohol, Stearylalkohol, Oleylalkohol, Elaidylalkohol, Linoleylalkohol, Linolenylalkohol, Nonadecylalkohol, Arachylalkohol, Gadoleylalkohol oder Arachidonalkohol, die als Alkali-, Erdalkali-, Ammonium-, Alkylammonium-, Alkanolamin- oder Glucammoniumsalz, bevorzugt als Natrium-, Kalium-, Alkanolamin-, Trialkylammonium-, Triethanolamin-, 2-Amino-1-butanol-, 2-Amino-2-methyl-1-propanol-, 2-Amino-2-methyl-1,3-propandiol-, 2-Amino-2-ethyl-1,3-propandiol- oder Tris-(hydroxymethyl)aminomethan-Salze sowie als Salze der basischen Aminosäuren Ornithin, Lysin, Arginin und/oder Histidin, vorliegen. Bevorzugt sind die Kaliumsalze der genannten Phosphorsäuremonoester. Besonders bevorzugt ist Dikaliummonocetylphosphat.

Weitere erfindungsgemäß bevorzugte Salze von $C_{12-20}$-Alkylphosphaten, die als anionischer Öl-in-Wasser-Emulgator einen ersten Teil des erfindungsgemäßen O/W-Emulgatorsystems bilden, sind ausgewählt aus den Diestern von Phosphorsäure mit Laurylalkohol, Tridecylalkohol, Isotridecylalkohol, Myristylalkohol, Pentadecylalkohol, Cetylalkohol, Palmitylalkohol, Isocetylalkohol, Isostearylalkohol, Stearylalkohol, Oleylalkohol, Elaidylalkohol, Linoleylalkohol, Linolenylalkohol, Nonadecylalkohol, Arachylalkohol, Gadoleylalkohol oder Arachidonalkohol, die als Alkali-, Erdalkali-, Ammonium-, Alkylammonium-, Alkanolamin- oder Glucammoniumsalz, bevorzugt als Natrium-, Kalium-, Alkanolamin-, Trialkylammonium-, Triethanolamin-, 2-Amino-1-butanol-, 2-Amino-2-methyl-1-propanol-, 2-Amino-2-methyl-1,3-propandiol-, 2-Amino-2-ethyl-1,3-propandiol- oder Tris-(hydroxymethyl)aminomethan-Salze sowie als Salze der basischen Aminosäuren Ornithin, Lysin, Arginin und/oder Histidin, vorliegen. Bevorzugt sind die Kaliumsalze der genannten Phosphorsäurediester. Besonders bevorzugt ist Kaliumdicetylphosphat.

Besonders bevorzugt sind Mischungen aus Mono-$C_{12-20}$-Alkylphosphaten und Di-$C_{12-20}$-Alkylphosphaten. Außerordentlich bevorzugt sind Mischungen aus Dikaliummonocetylphosphat und Kaliumdicetylphosphat.

Das mindestens eine Salz eines $C_{12-20}$-Alkylphosphats ist in den erfindungsgemäßen Zusammensetzungen in einer Gesamtmenge von 0,5 - 2,0 Gew.-%, bevorzugt 0,8 - 1,8 Gew.-%, besonders bevorzugt 1,2 - 1,5 Gew.-%, jeweils bezogen auf das Gesamtgewicht der Zusammensetzung, enthalten. Bevorzugte erfindungsgemäße Zusammensetzungen enthalten mindestens ein Salz von Cetylphosphat in einer Gesamtmenge von 0,5 - 2,0 Gew.-%, bevorzugt 0,8 - 1,8 Gew.-%, besonders bevorzugt 1,2 - 1,5 Gew.-%, jeweils bezogen auf das Gesamtgewicht der Zusammensetzung. Weitere bevorzugte erfindungsgemäße Zusammensetzungen enthalten eine Mischung aus Dikaliummonocetylphosphat und Kaliumdicetylphosphat in einer Gesamtmenge von 0,5 - 2,0 Gew.-%, bevorzugt 0,8 - 1,8 Gew.-%, besonders bevorzugt 1,2 - 1,5 Gew.-%, jeweils bezogen auf das Gesamtgewicht der Zusammensetzung.

Erfindungsgemäß bevorzugte $C_{14-20}$-Mono- oder Diacylglyceride, die den zweiten Teil des erfindungsgemäßen O/W-Emulgatorsystems bilden, sind ausgewählt aus Monomyristoylglycerid, Monopalmitoylglycerid, Monostearoylglycerid, Monoarachinoylglycerid, Dimyristoylglycerid, Dipalmitoylglycerid, Distearoylglycerid und Diarachinoylglycerid sowie aus Mischungen hiervon. Weitere erfindungsgemäß bevorzugte $C_{14-20}$-Mono- oder Diacylglyceridmischungen sind Glyceride gehärteter, das heißt, hydrierter, bevorzugt vollständig hydrierter, Fettsäuren natürlicher Öle. Erfindungsgemäß besonders bevorzugt sind gehärtete Palmölglyceride.

Das mindestens eine $C_{14-20}$-Mono- oder Diacylglycerid b) ist in den erfindungsgemäßen Zusammensetzungen in einer Gesamtmenge von 0,3 - 1,7 Gew.-%, bevorzugt 0,6 - 1,4 Gew.-%, besonders bevorzugt 0,9 - 1,1 Gew.-%, jeweils bezogen auf das Gesamtgewicht der Zusammensetzung, enthalten.

Bevorzugte erfindungsgemäße Zusammensetzungen enthalten mindestens eine Substanz, ausgewählt aus Monomyristoylglycerid, Monopalmitoylglycerid, Monostearoylglycerid, Monoarachinoylglycerid, Dimyristoylglycerid, Dipalmitoylglycerid, Distearoylglycerid und Diarachinoylglycerid sowie aus Mischungen hiervon, in einer Gesamtmenge von 0,3 - 1,7 Gew.-%, bevorzugt 0,6 - 1,4 Gew.-%, besonders bevorzugt 0,9 - 1,1 Gew.-%, jeweils bezogen auf das Gesamtgewicht der Zusammensetzung.

Weitere bevorzugte erfindungsgemäße Zusammensetzungen enthalten als $C_{14-20}$-Mono- oder Diacylglyceridmischung gehärtete Palmölglyceride in einer Gesamtmenge von 0,3 - 1,7 Gew.-%, bevorzugt 0,6 - 1,4 Gew.-%, besonders

bevorzugt 0,9 - 1,1 Gew.-%, jeweils bezogen auf das Gesamtgewicht der Zusammensetzung.

Weitere bevorzugte erfindungsgemäße Zusammensetzungen enthalten die mindestens eine Komponente a) und die mindestens eine Komponente b) im Gewichtsverhältnis 2/3 bis 3/2, bevorzugt 1/1 bis 3/2, besonders bevorzugt 6/5 bis 3/2.

Weitere bevorzugte erfindungsgemäße Zusammensetzungen enthalten, jeweils bezogen auf das Gesamtgewicht der Zusammensetzung, eine Mischung aus Dikaliummonocetylphosphat und Kaliumdicetylphosphat in einer Gesamtmenge von 0,5 - 2,0 Gew.-%, bevorzugt 0,8 - 1,8 Gew.-%, besonders bevorzugt 1,2 - 1,5 Gew.-%, weiterhin in einer Gesamtmenge von 0,3 - 1,7 Gew.-%, bevorzugt 0,6 - 1,4 Gew.-%, besonders bevorzugt 0,9 - 1,1 Gew.-%, mindestens eine Substanz, ausgewählt aus Monomyristoylglycerid, Monopalmitoylglycerid, Monostearoylglycerid, Monoarachinoylglycerid, Dimyristoylglycerid, Dipalmitoylglycerid, Distearoylglycerid und Diarachinoylglycerid sowie aus Mischungen hiervon.

Weitere bevorzugte erfindungsgemäße Zusammensetzungen enthalten, jeweils bezogen auf das Gesamtgewicht der Zusammensetzung, eine Mischung aus Dikaliummonocetylphosphat und Kaliumdicetylphosphat in einer Gesamtmenge von 0,5 - 2,0 Gew.-%, bevorzugt 0,8 - 1,8 Gew.-%, besonders bevorzugt 1,2 - 1,5 Gew.-%, weiterhin in einer Gesamtmenge von 0,3 - 1,7 Gew.-%, bevorzugt 0,6 - 1,4 Gew.-%, besonders bevorzugt 0,9 - 1,1 Gew.-%, mindestens eine Substanz, ausgewählt aus gehärteten Palmölglyceriden.

Eine erfindungsgemäß besonders bevorzugtes O/W-Emulgatorsystem, umfassend ein Gemisch aus Dikaliummonocetylphosphat und Kaliumdicetylphosphat mit gehärteten Palmölglyceriden, ist als Handelsprodukt "Emulsiphos 677660" (INCI: Potassium Cetyl Phosphate, Hydrogenated Palm Glycerides) von der Firma Symrise erhältlich.

Als weitere obligatorische Komponente, die zur Sensorikverbesserung, zum SPF-Boosting und darüber hinaus zur Stabilisierung der erfindungsgemäßen Zusammensetzungen beiträgt, sind 0,3 - 1 Gew.-% Behenylalkohol (Docosanol) enthalten, während lineare primäre Alkanole mit 12 bis 21 oder mehr als 22 Kohlenstoffatomen in einer Gesamtmenge von 0,03 bis 0,4 Gew.-% enthalten sind, wobei sich die Angaben in Gew.-% auf das Gesamtgewicht der Sonnenschutz-Zusammensetzung beziehen. Überraschend wurde festgestellt, dass sich mit einem höheren Gehalt an Behenylalkohol und anderen linearen primären Alkanolen mit 12 bis 21 und mehr als 22 Kohlenstoffatomen als 1,4 Gew.-%, wie beispielsweise 3 Gew.-%, zusammen mit den beanspruchten Komponenten a) und b) ein geringerer SPF und nur eine weniger bevorzugte Sensorik erzielt werden konnte und sich darüber hinaus Probleme bei der Herstellung lagerstabiler UV-Filter-haltiger Emulsionen ergaben, obwohl lineare Fettalkohole an sich bekannte Sensorikverbesserer und Emulsionsstabilisatoren darstellen.

Als weitere wesentliche Komponente enthalten die erfindungsgemäßen Zusammensetzungen n-Hexadecyl-n-nonanoat, n-Octadecyl-n-nonanoat oder eine Mischung hiervon in einer Gesamtmenge von 0,7 - 2 Gew.-%, bezogen auf das Gesamtgewicht der Sonnenschutz-Zusammensetzung. Überraschend wurde festgestellt, dass sich mit diesen Estern in einer Gesamtmenge von 0,7 - 2 Gew.-%, bezogen auf das Gesamtgewicht der Sonnenschutz-Zusammensetzung, Probleme bei der Herstellung sensorisch angenehmer und stabiler UV-Filter-haltiger Emulsionen mit hohem SPF erfolgreich lösen ließen. Bevorzugte erfindungsgemäße Sonnenschutz-Zusammensetzungen enthalten n-Hexadecyl-n-nonanoat, n-Octadecyl-n-nonanoat oder eine Mischung hiervon in einer Gesamtmenge von 1 - 1,5 Gew.-%, bezogen auf ihr Gesamtgewicht. Weitere bevorzugte erfindungsgemäße Zusammensetzungen enthalten, bezogen auf ihr Gesamtgewicht, eine Mischung von n-Hexadecyl-n-nonanoat und n-Octadecyl-n-nonanoat in einer Gesamtmenge von 0,7 - 2 Gew.-%, bevorzugt 1 - 1,5 Gew.-%.

Als weitere wesentliche Komponente enthalten die erfindungsgemäßen Zusammensetzungen, bezogen auf ihr Gesamtgewicht, 2 - 6 Gew.-%, bevorzugt 2,5 - 5 Gew.-%, besonders bevorzugt 3 - 4 Gew.-%, 2-Ethylhexyl-3,5,5-trimethylhexanoat. Überraschend wurde festgestellt, dass sich mit diesem Öl in der vorstehend angegebenen Menge in Kombination mit n-Hexadecyl-n-nonanoat, n-Octadecyl-n-nonanoat oder einer Mischung hiervon in einer Gesamtmenge von 0,7 - 2 Gew.-% Probleme bei der Herstellung sensorisch angenehmer und stabiler UV-Filter-haltiger Emulsionen mit hohem SPF erfolgreich lösen ließen.

"Normalbedingungen" sind im Sinne der vorliegenden Anmeldung eine Temperatur von 20 °C und ein Druck von 1013,25 mbar. Schmelzpunktangaben beziehen sich ebenfalls auf einen Druck von 1013,25 mbar.

Überraschend wurde gefunden, dass unter Normalbedingungen feste Ester, die aus linearen Carbonsäuren mit 7 oder 8 Kohlenstoffatomen und linearen Alkanolen mit 16 oder 18 Kohlenstoffatomen gebildet sind, also strukturell sehr ähnlich sind zu n-Hexadecyl-n-nonanoat und n-Octadecyl-n-nonanoat, zu Rezepturen mit einem vergleichsweise geringeren SPF und einer weniger bevorzugten Sensorik führten und sich darüber hinaus Probleme bei der Herstellung lagerstabiler UV-Filter-haltiger Emulsionen ergaben.

Erfindungsgemäß bevorzugt sind daher Sonnenschutz-Zusammensetzungen, die, bezogen auf ihr Gesamtgewicht, unter Normalbedingungen feste Ester, die aus linearen Carbonsäuren mit 1 - 8 Kohlenstoffatomen und linearen Alkanolen mit 1 - 18 Kohlenstoffatomen gebildet sind, in einer Gesamtmenge von 0 bis 0,6 Gew.-% enthalten sind.

Bevorzugte erfindungsgemäße kosmetische Zusammensetzungen weisen bei 20°C einen pH-Wert im Bereich von 5,7 - 7,3, besonders bevorzugt einen pH-Wert im Bereich von 6,0 bis 6,7, außerordentlich bevorzugt im Bereich von 6,2 bis 6,4, auf.

Erfindungsgemäße kosmetische Zusammensetzungen enthalten, bezogen auf ihr Gesamtgewicht, mindestens 15 Gew.-% mindestens einer in der Ölphase oder der Wasserphase gelösten organischen UV-Filtersubstanz, darunter 2-Cyano-3,3-phenylzimtsäure-2-ethylhexylester, 4-(tert.-Butyl)-4'-methoxydibenzoylmethan und mindestens eine in der Wasserphase gelöste organische UV-Filtersubstanz, die ausgewählt ist aus Phenylen-1,4-bis-(2-benzimidazyl)-3,3'-5,5'-tetrasulfonsäure, 2-Phenylbenzimidazol-5-sulfonsäure und den Salzen dieser Säuren sowie aus Mischungen der genannten Sulfonsäurederivate, dadurch gekennzeichnet, dass die mindestens eine in der Ölphase gelöste organische UV- Filtersubstanz 3,3,5-Trimethylcyclohexylsalicylat (Homosalat) umfasst. Mit den obligatorischen UV-Filtern in den beanspruchten Mengen lassen sich Sonnenschutzzusammensetzungen mit hohem Lichtschutzfaktor herstellen, sofern man sie in die richtige Emulsionsbasis einarbeitet. Problematisch für die Lagerstabilität der Emulsionen können insbesondere die Polarität dieser UV-Filter und ihr Löslichkeitsverhalten sein. Weiterhin ist es schwierig, mit den obligatorischen UV-Filtern in den beanspruchten Mengen Sonnenschutzzusammensetzungen zu formulieren, die bei und nach der Applikation auf die Haut eine Sensorik aufweisen, die von der Mehrzahl der Verbraucher tatsächlich bevorzugt wird. Erfindungsgemäß bevorzugte kosmetische Zusammensetzungen enthalten, jeweils bezogen auf ihr Gesamtgewicht, bis zu 40 Gew.-%, bevorzugt 17-30 Gew.-%, besonders bevorzugt 19-27 Gew.-%, außerordentlich bevorzugt 21 - 25 Gew.-%, mindestens einer in der Ölphase oder der Wasserphase gelösten organischen UV-Filtersubstanz, darunter 2-Cyano-3,3-phenylzimtsäure-2-ethylhexylester, 4-(tert.-Butyl)-4'-methoxydibenzoylmethan und mindestens eine in der Wasserphase gelöste organische UV-Filtersubstanz, die ausgewählt ist aus Phenylen-1,4-bis-(2-benzimidazyl)-3,3'-5,5'-tetrasulfonsäure, 2-Phenylbenzimidazol-5-sulfonsäure und den Salzen dieser Säuren sowie aus Mischungen der genannten Sulfonsäurederivate.

Bevorzugte erfindungsgemäße kosmetische Zusammensetzungen enthalten 2-Cyano-3,3-phenylzimtsäure-2-ethylhexylester als in der Ölphase gelöste organische UV-Filtersubstanz. Der öllösliche UVB-Filter 2-Cyano-3,3-phenylzimtsäure-2-ethylhexylester (= Ethylhexyl-2-cyano-3,3-diphenylacrylat hat die INCI-Bezeichnung Octocrylene. Octocrylene ist bevorzugt in einer Gesamtmenge von 0,5 bis 15 Gew.-%, besonders bevorzugt 3 bis 13 Gew.-%, insbesondere 5 bis 11 Gew.-% und außerordentlich bevorzugt 8 - 10 Gew.-%, enthalten, jeweils bezogen auf das Gesamtgewicht der erfindungsgemäßen Zusammensetzung.

Weitere bevorzugte erfindungsgemäße kosmetische Zusammensetzungen enthalten 4-tert-Butyl-4'-Methoxydibenzoylmethan als in der Ölphase gelöste organische UV-Filtersubstanz. 4-tert-Butyl-4'-Methoxydibenzoylmethan mit der INCI-Bezeichnung Butyl Methoxydibenzoylmethane (auch als 1-(4'-tert-Butylphenyl)-3-(4'-methoxyphenyl)propan-1,3-dion bezeichnet) ist ein öllöslicher UVA-Lichtschutzfilter.

Bevorzugte erfindungsgemäße Zusammensetzungen enthalten 4-tert-Butyl-4'-Methoxydibenzoylmethan in einer Gesamtmenge von 0,5 bis 10 Gew.-%, bevorzugt 1 bis 8 Gew.-%, besonders bevorzugt 2 bis 5 Gew.-% und außerordentlich bevorzugt 3 - 4 Gew.-%, jeweils bezogen auf das Gesamtgewicht der erfindungsgemäßen Zusammensetzung.

Besonders bevorzugte erfindungsgemäße Zusammensetzungen enthalten 4-tert-Butyl-4'-Methoxydibenzoylmethan und 2-Cyano-3,3-phenylzimtsäure-2-ethylhexylester. Erfindungsgemäße kosmetische Zusammensetzungen enthalten 3,3,5-Trimethylcyclohexylsalicylat. 3,3,5-Trimethylcyclohexylsalicylat, auch 3,3,5-Trimethylcyclohexyl-2-hydroxybenzoat oder Homomenthylsalicylat genannt, mit der INCI-Bezeichnung "Homosalate", ist ein öllöslicher UVB-Filter und z.B. als Neo Heliopan HMS von Symrise erhältlich. Bevorzugte erfindungsgemäße Öl-in-Wasser-Emulsionen enthalten 3,3,5-Trimethylcyclohexylsalicylat in einer Gesamtmenge von 0,5 - 10 Gew.-%, bevorzugt 2 - 9 Gew.-%, besonders bevorzugt 4 - 8 Gew.-%, außerordentlich bevorzugt 5,5 - 7 Gew.-%, jeweils bezogen auf das Gesamtgewicht der erfindungsgemäßen Zusammensetzung. 3,3,5-Trimethylcyclohexylsalicylat hat gegenüber vielen anderen öllöslichen UVB-Filtern, insbesondere gegenüber anderen Salicylsäureester-Filtern, wie 2-Ethylhexylsalicylat oder 4-Isopropylbenzylsalicylat, diverse Vorteile: bei gleicher Konzentration erzielt man einen höheren Lichtschutzfaktor; weiterhin weisen Emulsionen mit diesem Filter im Vergleich zu anderen öllöslichen Salicylsäureester-Filtern, wie 2-Ethylhexylsalicylat, eine bessere Stabilität auf, insbesondere bei längerer Lagerdauer und/oder bei Lagerung bei erhöhten Temperaturen (beispielsweise 45°C). Weiterhin weisen Emulsionen mit diesem Filter im Vergleich zu anderen öllöslichen Salicylsäureester-Filtern, wie 2-Ethylhexylsalicylat oder 4-Isopropylbenzylsalicylat, bessere haptische Eigenschaften auf, insbesondere eine geringere Klebrigkeit und ein weniger stumpfes Hautgefühl.

In einer bevorzugten Ausführungsform sind die erfindungsgemäßen Zusammensetzungen frei von 2-Ethylhexylsalicylat und frei von 4-Isopropylbenzylsalicylat.

In einer weiteren bevorzugten Ausführungsform sind die erfindungsgemäßen Zusammensetzungen frei von Polysilicone-15.

Aufgrund des angenehmeren Hautgefühls bevorzugen viele Verbraucher Öl-in-Wasser-Emulsionen gegenüber Wasser-in-Öl-Emulsionen. Da die äußere Wasserphase in solchen Emulsionen häufig einen höheren Gewichtsanteil hat als die dispergierte Öl-/Fettphase, ist es vorteilhaft, die UV-Filtersubstanzen auf beide Phasen zu verteilen und also neben den öllöslichen organischen UV-Filtern, bevorzugt 2-Cyano-3,3-phenylzimtsäure-2-ethylhexylester, 4-(tert.-Butyl)-4'-methoxydibenzoylmethan und 3,3,5-Trimethylcyclohexylsalicylat (Homosalate) auch mindestens eine in der Wasserphase gelöste organische UV-Filtersubstanz einzuarbeiten.

Die erfindungsgemäßen kosmetischen Zusammensetzungen enthalten demnach mindestens eine in der Wasserphase gelöste organische UV-Filtersubstanz, die ausgewählt ist aus Phenylen-1,4-bis-(2-benzimidazyl)-3,3'-5,5'-tetrasulfonsäure, 2-Phenylbenzimidazol-5-sulfonsäure und den Salzen dieser Säuren sowie aus Mischungen der genannten Sulfonsäurederivate.

Bevorzugte erfindungsgemäße kosmetische Zusammensetzungen enthalten mindestens eine in der Wasserphase gelöste organische UV-Filtersubstanz in einer Gesamtmenge von 0,5 Gew.-% bis 15 Gew.-%, bevorzugt 1 bis 10 Gew.-%, besonders bevorzugt 2 bis 5 Gew.-% und außerordentlich bevorzugt 3 - 4 Gew.-%, jeweils bezogen auf das Gesamtgewicht der erfindungsgemäßen Zusammensetzung.

Phenylen-1,4-bis-(2-benzimidazyl)-3,3'-5,5'-tetrasulfonsäure und ihre Salze, insbesondere die Alkali-, Erdalkali-, Ammonium-, Alkylammonium-, Alkanolamin- und Glucammoniumsalze, bevorzugt die entsprechenden Natrium-, Kalium-, Trialkylammonium- oder Triethanolamin-, Lysin-, Arginin-, Histidin- und Ornithin-Salze, wirken als UV-A-Filter. Ebenfalls erfindungsgemäß geeignet, wenn auch weniger bevorzugt, sind die Salze dieser Säure mit Amino-$C_1$-$C_6$-Alkanolen und Amino-$C_1$-$C_6$-Alkandiolen, insbesondere mit 2-Amino-2-methylpropan-1-ol, 2-Amino-2-methylpropan-1,3-diol, 2-Aminopropan-1-ol, 3-Aminopropan-1-ol, 1-Aminopropan-2-ol (MIPA) und 2-Amino-2-(hydroxymethyl)propan-1,3-diol (TRIS) sowie Mischungen hiervon.

Erfindungsgemäß bevorzugt liegt ein Anteil der Phenylen-1,4-bis-(2-benzimidazyl)-3,3'-5,5'-tetrasulfonsäure oder der 2-Phenylbenzimidazol-5-sulfonsäure als Salz von mindestens einer basischen Aminosäure, ausgewählt aus Lysin, Arginin, Histidin und Ornithin, vor. Bevorzugt beträgt dieser Anteil mindestens 30 Mol-%, besonders bevorzugt mindestens 50 Mol-%, außerordentlich bevorzugt mindestens 70 Mol-%, jeweils bezogen auf den Gesamtgehalt an Benzimidazolsulfonsäure-Derivat. Eine bevorzugte Aminosäure ist Arginin. Besonders bevorzugt beträgt der Anteil an Arginin-Salz mindestens 30 Mol-%, besonders bevorzugt mindestens 50 Mol.-%, außerordentlich bevorzugt mindestens 70 Mol-%, jeweils bezogen auf den Gesamtgehalt an Benzimidazolsulfonsäure-Derivat.

Neben dem Salz von mindestens einer basischen Aminosäure, ausgewählt aus Lysin, Arginin, Histidin und Ornithin, können weitere Salze der Phenylen-1,4-bis-(2-benzimidazyl)-3,3'-5,5'-tetrasulfonsäure oder der 2-Phenylbenzimidazol-5-sulfonsäure enthalten sein. Als weiteres Salz bevorzugt sind die Alkali-, Erdalkali-, Ammonium-, Alkylammonium-, Alkanolamin- und Glucammoniumsalze, bevorzugt die entsprechenden Natrium-, Kalium-, Trialkylammonium- oder Triethanolamin-Salze. Außerordentlich bevorzugt ist das Phenylen-1,4-bis-(2-benzimidazyl)-3,3'-5,5'-tetrasulfonsäure-bis-natriumsalz mit der INCI-Bezeichnung "Disodium Phenyl Dibenzimidazole Tetrasulfonate" (CAS-Nr.: 180898-37-7), das beispielsweise unter der Handelsbezeichnung Neo Heliopan AP von Symrise erhältlich ist. Weitere bevorzugte wasserlösliche organische UV-Filtersubstanzen sind die 2-Phenylbenzimidazol-5-sulfonsäure (UV-B-Filter, INCI-Bezeichnung "Phenylbenzimidazole sulfonic acid" (CAS.-Nr. 27503-81-7)) und ihre Salze, insbesondere die Alkali-, Erdalkali-, Ammonium-, Alkylammonium-, Alkanolamin- und Glucammoniumsalze, bevorzugt die entsprechenden Natrium-, Kalium-, Trialkylammonium- oder Triethanolamin-Salze, weiterhin die Salze dieser Säure mit mindestens einer basischen Aminosäure, ausgewählt aus Lysin, Arginin, Histidin und Ornithin, insbesondere mit Arginin, insbesondere aber Mischungen der Salze der 2-Phenylbenzimidazol-5-sulfonsäure mit Natriumionen und mit Arginin.

In einer besonders bevorzugten Ausführungsform der Erfindung ist das mindestens eine Salz des wasserlöslichen organischen UV-Filters, der ausgewählt ist aus Phenylen-1,4-bis-(2-benzimidazyl)-3,3'-5,5'-tetrasulfonsäure und 2-Phenylbenzimidazol-5-sulfonsäure, ausgewählt aus den Natrium-, Kalium- und Triethanolamin-Salzen, insbesondere aus dem Phenylbenzimidazolsulfonsäure-Natrium-Salz und dem Phenylen-1,4-bis-(2-benzimidazyl)-3,3'-5,5'-tetrasulfonsäure-bis-natriumsalz, sowie Mischungen hiervon.

In einer weiteren besonders bevorzugten Ausführungsform der Erfindung ist das mindestens eine Salz des wasserlöslichen organischen UV-Filters, der ausgewählt ist aus Phenylen-1,4-bis-(2-benzimidazyl)-3,3'-5,5'-tetrasulfonsäure und 2-Phenylbenzimidazol-5-sulfonsäure, ausgewählt aus den Salzen dieser Säuren mit mindestens einer basischen Aminosäure, die ausgewählt ist aus Arginin, Lysin, Histidin und Ornithin, insbesondere aus den Salzen der Phenylbenzimidazolsulfonsäure und des Phenylen-1,4-bis-(2-benzimidazyl)-3,3'-5,5'-tetrasulfonsäure-bis-natriumsalzes mit Natrium und mit Arginin als Gegenionen. Diese bevorzugten Salze können in besonders bevorzugter Ausführungsform in Kombination mit Alkali-, Erdalkali-, Ammonium-, Alkylammonium-, Alkanolamin- und Glucammoniumsalzen, bevorzugt mit Natrium-, Kalium-, Trialkylammonium- oder Triethanolamin-Salzen vorliegen. Besonders bevorzugt sind das Phenylbenzimidazolsulfonsäure-Arginin-Salz, das Phenylen-1,4-bis-(2-benzimidazyl)-3,3'-5,5'-tetrasulfonsäure-bis-natrium-bis-arginin-Salz sowie Mischungen hiervon.

Bevorzugte erfindungsgemäße Zusammensetzungen enthalten Phenylen-1,4-bis-(2-benzimidazyl)-3,3'-5,5'-tetrasulfonsäure, 2-Phenylbenzimidazol-5-sulfonsäure und/oder deren Salze in einer Gesamtmenge von 0,5 bis 15 Gew.-%, bevorzugt 1 bis 10 Gew.-%, besonders bevorzugt 2 bis 5 Gew.-% und außerordentlich bevorzugt 3 - 4 Gew.-%, jeweils bezogen auf das Gesamtgewicht der erfindungsgemäßen Zusammensetzung.

Die vorstehenden und die folgenden Gewichtsangaben beziehen sich dabei, unabhängig von der Form, in der das Benzimidazolsulfonsäure-Derivat vorliegt (Salz, freie Säure), auf das Gewicht des Benzimidazolsulfonsäure-Derivats in der Säureform.

Weitere bevorzugte erfindungsgemäße Zusammensetzungen enthalten 2-Phenylbenzimidazol-5-sulfonsäure oder ein Salz hiervon in einer Gesamtmenge von 0,5 bis 8 Gew.-%, bevorzugt 1 bis 6 Gew.-%, besonders bevorzugt

1,5 bis 4 Gew.-% und außerordentlich bevorzugt 2 - 3 Gew.-%, jeweils bezogen auf das Gesamtgewicht der erfindungsgemäßen Zusammensetzung.

Weitere bevorzugte erfindungsgemäße Zusammensetzungen enthalten Phenylen-1,4-bis-(2-benzimidazyl)-3,3'-5,5'-tetrasulfonsäure oder ein Salz hiervon in einer Gesamtmenge von 0,1 bis 10 Gew.-%, bevorzugt 0,5 bis 8 Gew.-%, besonders bevorzugt 0,8 bis 5 Gew.-% und außerordentlich bevorzugt 0,9 - 2 Gew.-%, jeweils bezogen auf das Gesamtgewicht der erfindungsgemäßen Zusammensetzung.

In weiteren bevorzugten erfindungsgemäßen Zusammensetzungen beträgt die Gesamtmenge aller Benzimidazolsulfonsäure-Derivate 0,5 bis 15 Gew.-%, bevorzugt 1 bis 10 Gew.-%, besonders bevorzugt 2 bis 5 Gew.-% und außerordentlich bevorzugt 3 - 4 Gew.-%, jeweils bezogen auf das Gesamtgewicht der erfindungsgemäßen Zusammensetzung.

Weitere bevorzugte erfindungsgemäße Zusammensetzungen enthalten Phenylen-1,4-bis-(2-benzimidazyl)-3,3'-5,5'-tetrasulfonsäure und 2-Phenylbenzimidazol-5-sulfonsäure oder Salze hiervon in einer Gesamtmenge von 0,5 - 15 Gew.-%, bevorzugt 1 - 10 Gew.-%, besonders bevorzugt 2 - 5 Gew.-% und außerordentlich bevorzugt 3 - 4 Gew.-%, jeweils bezogen auf das Gesamtgewicht der erfindungsgemäßen Zusammensetzung.

Weitere bevorzugte erfindungsgemäße Zusammensetzungen enthalten a) 2-Cyano-3,3-phenylzimtsäure-2-ethylhexylester, b) 4-(tert.-Butyl)-4'-methoxydibenzoylmethan, c) 3,3,5-Trimethylcyclohexylsalicylat und d) 2-Phenylbenzimidazol-5-sulfonsäure-Natriumsalz.

Weitere bevorzugte erfindungsgemäße Zusammensetzungen enthalten a) 2-Cyano-3,3-phenylzimtsäure-2-ethylhexylester, b) 4-(tert.-Butyl)-4'-methoxydibenzoylmethan, c) 3,3,5-Trimethylcyclohexylsalicylat und d) 2-Phenylbenzimidazol-5-sulfonsäure-Argininsalz.

Weitere bevorzugte erfindungsgemäße Zusammensetzungen enthalten a) 2-Cyano-3,3-phenylzimtsäure-2-ethylhexylester, b) 4-(tert.-Butyl)-4'-methoxydibenzoylmethan, c) 3,3,5-Trimethylcyclohexylsalicylat und d) Dinatriumphenylbenzimidazoltetrasulfonat.

Weitere bevorzugte erfindungsgemäße Zusammensetzungen enthalten ein Gemisch aus Dikaliummonocetylphosphat und Kaliumdicetylphosphat mit gehärteten Palmölglyceriden, weiterhin a) 2-Cyano-3,3-phenylzimtsäure-2-ethylhexylester, b) 4-(tert.-Butyl)-4'-methoxydibenzoylmethan, c) 3,3,5-Trimethylcyclohexylsalicylat, d)i) 2-Phenylbenzimidazol-5-sulfonsäure-Argininsalz und d)ii) Dinatriumphenylbenzimidazoltetrasulfonat.

Weitere bevorzugte erfindungsgemäße Zusammensetzungen enthalten ein Gemisch aus Dikaliummonocetylphosphat und Kaliumdicetylphosphat mit gehärteten Palmölglyceriden in einer Gesamtmenge von 0,5 - 4 Gew.-%, besonders bevorzugt 1 - 3,7 Gew.-%, insbesondere 2 - 3 Gew.-% und außerordentlich bevorzugt 2 - 2,5 Gew.-%, weiterhin 2-Cyano-3,3-phenylzimtsäure-2-ethylhexylester in einer Gesamtmenge von 0,5 - 15 Gew.-%, besonders bevorzugt 3 - 13 Gew.-%, insbesondere 5 - 11 Gew.-% und außerordentlich bevorzugt 8 - 10 Gew.-%, 4-tert-Butyl-4'-methoxydibenzoylmethan in einer Gesamtmenge von 0,5 - 10 Gew.-%, bevorzugt 1 - 8 Gew.-%, besonders bevorzugt 2 - 5 Gew.-% und außerordentlich bevorzugt 3 - 4 Gew.-%, 3,3,5-Trimethylcyclohexylsalicylat in einer Gesamtmenge von 0,5 - 10 Gew.-%, bevorzugt 2 - 9 Gew.-%, besonders bevorzugt 4 - 8 Gew.-%, außerordentlich bevorzugt 5,5 - 7 Gew.-%, und das Argininsalz der 2-Phenylbenzimidazol-5-sulfonsäure in einer Gesamtmenge von 0,5 - 8 Gew.-%, bevorzugt 1 - 6 Gew.-%, besonders bevorzugt 1,5 - 4 Gew.-% und außerordentlich bevorzugt 2 - 3 Gew.-%, jeweils bezogen auf das Gesamtgewicht der erfindungsgemäßen Zusammensetzung.

Weitere bevorzugte erfindungsgemäße Zusammensetzungen enthalten ein Gemisch aus Dikaliummonocetylphosphat und Kaliumdicetylphosphat mit gehärteten Palmölglyceriden in einer Gesamtmenge von 0,5 - 4 Gew.-%, besonders bevorzugt 1 - 3,7 Gew.-%, insbesondere 2 - 3 Gew.-% und außerordentlich bevorzugt 2 - 2,5 Gew.-%, weiterhin 2-Cyano-3,3-phenylzimtsäure-2-ethylhexylester in einer Gesamtmenge von 0,5 - 15 Gew.-%, besonders bevorzugt 3 bis 13 Gew.-%, insbesondere 5 bis 11 Gew.-% und außerordentlich bevorzugt 8 - 10 Gew.-%, 4-tert-Butyl-4'-methoxydibenzoylmethan in einer Gesamtmenge von 0,5 - 10 Gew.-%, bevorzugt 1 - 8 Gew.-%, besonders bevorzugt 2 - 5 Gew.-% und außerordentlich bevorzugt 3 - 4 Gew.-%, 3,3,5-Trimethylcyclohexylsalicylat in einer Gesamtmenge von 0,5 - 10 Gew.-%, bevorzugt 2 - 9 Gew.-%, besonders bevorzugt 4 - 8 Gew.-%, außerordentlich bevorzugt 5,5 - 7 Gew.-%, und Dinatriumphenylbenzimidazoltetrasulfonat in einer Gesamtmenge von 0,1 - 10 Gew.-%, bevorzugt 0,5 - 8 Gew.-%, besonders bevorzugt 0,8 - 5 Gew.-% und außerordentlich bevorzugt 0,9 - 2 Gew.-%, jeweils bezogen auf das Gesamtgewicht der erfindungsgemäßen Zusammensetzung.

Weitere bevorzugte erfindungsgemäße Zusammensetzungen enthalten ein Gemisch aus Dikaliummonocetylphosphat und Kaliumdicetylphosphat mit gehärteten Palmölglyceriden in einer Gesamtmenge von 0,5 - 4 Gew.-%, besonders bevorzugt 1 - 3,7 Gew.-%, insbesondere 2 - 3 Gew.-% und außerordentlich bevorzugt 2 - 2,5 Gew.-%, weiterhin 2-Cyano-3,3-phenylzimtsäure-2-ethylhexylester in einer Gesamtmenge von 0,5 - 15 Gew.-%, besonders bevorzugt 3 - 13 Gew.-%, insbesondere 5 - 11 Gew.-% und außerordentlich bevorzugt 8 - 10 Gew.-%, 4-tert-Butyl-4'-methoxydibenzoylmethan in einer Gesamtmenge von 0,5 - 10 Gew.-%, bevorzugt 1 - 8 Gew.-%, besonders bevorzugt 2 - 5,5 Gew.-% und außerordentlich bevorzugt 3 - 4 Gew.-%, 3,3,5-Trimethylcyclohexylsalicylat in einer Gesamtmenge von 0,5 - 10 Gew.-%, bevorzugt 2 - 9 Gew.-%, besonders bevorzugt 4 - 8 Gew.-%, außerordentlich bevorzugt 5,5 - 7 Gew.-%, das Argininsalz der 2-Phenylbenzimidazol-5-sulfonsäure in einer Gesamtmenge von 0,5 - 8 Gew.-%, bevorzugt

1 - 6 Gew.-%, besonders bevorzugt 1,5 - 4 Gew.-% und außerordentlich bevorzugt 2 - 3 Gew.-%, und Dinatriumphenylbenzimidazoltetrasulfonat in einer Gesamtmenge von 0,1 - 10 Gew.-%, bevorzugt 0,5 - 8 Gew.-%, besonders bevorzugt 0,8 - 5 Gew.-% und außerordentlich bevorzugt 0,9 - 2 Gew.-%, jeweils bezogen auf das Gesamtgewicht der erfindungsgemäßen Zusammensetzung.

Weitere bevorzugte erfindungsgemäße Zusammensetzungen enthalten die UV-Filter a), b), c) und d) in einem Gewichtsverhältnis zueinander von a) : b) : c) : d) wie (3 - 3,5) : (1 - 1,5) : (2 - 2,5) : (0,8 - 1,2).

Weitere bevorzugte erfindungsgemäße Zusammensetzungen enthalten die UV-Filter a), b), c) und d) in einem Gewichtsverhältnis zueinander von a) : b) : c) : d) wie (3,2 - 3,4) : (1,2 - 1,4) : (2,2 - 2,4) : (0,9 - 1,1), bevorzugt 3,3 : 1,3 : 2,3 : 1.

Weitere bevorzugte erfindungsgemäße Zusammensetzungen enthalten ein Gemisch aus Dikaliummonocetylphosphat und Kaliumdicetylphosphat mit gehärteten Palmölglyceriden und weiterhin 3,3,5-Trimethylcyclohexylsalicylat und weisen einen pH-Wert bei 20°C von 5,7 - 7,3, bevorzugt 6,0 - 6,7, besonders bevorzugt 6,2 bis 6,4, auf.

Weitere bevorzugte erfindungsgemäße Zusammensetzungen enthalten ein Gemisch aus Dikaliummonocetylphosphat und Kaliumdicetylphosphat mit gehärteten Palmölglyceriden, weiterhin a) 2-Cyano-3,3-phenylzimtsäure-2-ethylhexylester, b) 4-(tert.-Butyl)-4'-methoxydibenzoylmethan, c) 3,3,5-Trimethylcyclohexylsalicylat, d)i) 2-Phenylbenzimidazol-5-sulfonsäure-Argininsalz und d)ii) Dinatriumphenylbenzimidazoltetrasulfonat und weisen einen pH-Wert bei 20°C von 5,7 - 7,3, bevorzugt 6,0 - 6,7, besonders bevorzugt 6,2 bis 6,4, auf.

Weitere bevorzugte erfindungsgemäße Zusammensetzungen enthalten ein Gemisch aus Dikaliummonocetylphosphat und Kaliumdicetylphosphat mit gehärteten Palmölglyceriden in einer Gesamtmenge von 0,5 - 4 Gew.-%, besonders bevorzugt 1 - 3,7 Gew.-%, insbesondere 2 - 3 Gew.-% und außerordentlich bevorzugt 2 - 2,5 Gew.-%, weiterhin 2-Cyano-3,3-phenylzimtsäure-2-ethylhexylester in einer Gesamtmenge von 0,5 - 15 Gew.-%, besonders bevorzugt 3 - 13 Gew.-%, insbesondere 5 - 11 Gew.-% und außerordentlich bevorzugt 8 - 10 Gew.-%, 4-tert-Butyl-4'-methoxydibenzoylmethan in einer Gesamtmenge von 0,5 - 10 Gew.-%, bevorzugt 1 - 8 Gew.-%, besonders bevorzugt 2 - 5 Gew.-% und außerordentlich bevorzugt 3 - 4 Gew.-%, 3,3,5-Trimethylcyclohexylsalicylat in einer Gesamtmenge von 0,5 - 10 Gew.-%, bevorzugt 2 - 9 Gew.-%, besonders bevorzugt 4 - 8 Gew.-%, außerordentlich bevorzugt 5,5 - 7 Gew.-%, und das Argininsalz der 2-Phenylbenzimidazol-5-sulfonsäure in einer Gesamtmenge von 0,5 - 8 Gew.-%, bevorzugt 1 - 6 Gew.-%, besonders bevorzugt 1,5 - 4 Gew.-% und außerordentlich bevorzugt 2 - 3 Gew.-%, jeweils bezogen auf das Gesamtgewicht der erfindungsgemäßen Zusammensetzung, und weisen einen pH-Wert bei 20°C von 5,7 - 7,3, bevorzugt 6,0 - 6,7, besonders bevorzugt 6,2 bis 6,4, auf.

Weitere bevorzugte erfindungsgemäße Zusammensetzungen enthalten ein Gemisch aus Dikaliummonocetylphosphat und Kaliumdicetylphosphat mit gehärteten Palmölglyceriden in einer Gesamtmenge von 0,5 - 4 Gew.-%, besonders bevorzugt 1 - 3,7 Gew.-%, insbesondere 2 - 3 Gew.-% und außerordentlich bevorzugt 2 - 2,5 Gew.-%, weiterhin 2-Cyano-3,3-phenylzimtsäure-2-ethylhexylester in einer Gesamtmenge von 0,5 - 15 Gew.-%, besonders bevorzugt 3 bis 13 Gew.-%, insbesondere 5 bis 11 Gew.-% und außerordentlich bevorzugt 8 - 10 Gew.-%, 4-tert-Butyl-4'-methoxydibenzoylmethan in einer Gesamtmenge von 0,5 - 10 Gew.-%, bevorzugt 1 - 8 Gew.-%, besonders bevorzugt 2 - 5 Gew.-% und außerordentlich bevorzugt 3 - 4 Gew.-%, 3,3,5-Trimethylcyclohexylsalicylat in einer Gesamtmenge von 0,5 - 10 Gew.-%, bevorzugt 2 - 9 Gew.-%, besonders bevorzugt 4 - 8 Gew.-%, außerordentlich bevorzugt 5,5 - 7 Gew.-%, und Dinatriumphenylbenzimidazoltetrasulfonat in einer Gesamtmenge von 0,1 - 10 Gew.-%, bevorzugt 0,5 - 8 Gew.-%, besonders bevorzugt 0,8 - 5 Gew.-% und außerordentlich bevorzugt 0,9 - 2 Gew.-%, jeweils bezogen auf das Gesamtgewicht der erfindungsgemäßen Zusammensetzung, und weisen einen pH-Wert bei 20°C von 5,7 - 7,3, bevorzugt 6,0 - 6,7, besonders bevorzugt 6,2 bis 6,4, auf.

Weitere bevorzugte erfindungsgemäße Zusammensetzungen enthalten ein Gemisch aus Dikaliummonocetylphosphat und Kaliumdicetylphosphat mit gehärteten Palmölglyceriden in einer Gesamtmenge von 0,5 - 4 Gew.-%, besonders bevorzugt 1 - 3,7 Gew.-%, insbesondere 2 - 3 Gew.-% und außerordentlich bevorzugt 2 - 2,5 Gew.-%, weiterhin 2-Cyano-3,3-phenylzimtsäure-2-ethylhexylester in einer Gesamtmenge von 0,5 - 15 Gew.-%, besonders bevorzugt 3 - 13 Gew.-%, insbesondere 5 - 11 Gew.-% und außerordentlich bevorzugt 8 - 10 Gew.-%, 4-tert-Butyl-4'-methoxydibenzoylmethan in einer Gesamtmenge von 0,5 - 10 Gew.-%, bevorzugt 1 - 8 Gew.-%, besonders bevorzugt 2 - 5 Gew.-% und außerordentlich bevorzugt 3 - 4 Gew.-%, 3,3,5-Trimethylcyclohexylsalicylat in einer Gesamtmenge von 0,5 - 10 Gew.-%, bevorzugt 2 - 9 Gew.-%, besonders bevorzugt 4 - 8 Gew.-%, außerordentlich bevorzugt 5,5 - 7 Gew.-%, das Argininsalz der 2-Phenylbenzimidazol-5-sulfonsäure in einer Gesamtmenge von 0,5 - 8 Gew.-%, bevorzugt 1 - 6 Gew.-%, besonders bevorzugt 1,5 - 4 Gew.-% und außerordentlich bevorzugt 2 - 3 Gew.-%, und Dinatriumphenylbenzimidazoltetrasulfonat in einer Gesamtmenge von 0,1 - 10 Gew.-%, bevorzugt 0,5 - 8 Gew.-%, besonders bevorzugt 0,8 - 5 Gew.-% und außerordentlich bevorzugt 0,9 - 2 Gew.-%, jeweils bezogen auf das Gesamtgewicht der erfindungsgemäßen Zusammensetzung, und weisen einen pH-Wert bei 20°C von 5,7 - 7,3, bevorzugt 6,0 - 6,7, besonders bevorzugt 6,2 bis 6,4, auf.

Derartige Zusammensetzungen lösen überraschend insbesondere die Aufgabe, eine Sonnenschutzzusammensetzung bereitzustellen, die die Anforderung der EU-Kommissions-Empfehlung 2006/647/EC, Abschnitt 3, Absatz 10b, erfüllt und ein Verhältnis von Lichtschutzfaktor zu UV-A-Schutzfaktor unter 3 aufweist.

Bevorzugte erfindungsgemäße kosmetische Zusammensetzungen enthalten kein Polysilicone-15. Die Substanz mit der INCI-Bezeichnung Polysilicone-15 wird auch als 3-(4-(2,2-Bis-Ethoxycarbonylvinyl)-phenoxy)propenyl)-methoxysiloxan/Dimethylsiloxan-Copolymer, Dimethicodiethylbenzalmalonat, Diethylbenzylidene Malonate Dimethicone oder Diethylmalonylbenzylidene Oxypropene Dimethicone bezeichnet und ist unter dem Handelsnamen Parsol® SLX (INCI-Bezeichnung Dimethicodiethylbenzal malonate (CAS-Nr. 207574-74-1)) von DSM erhältlich. Die chemische Struktur ist beispielsweise in EP 709080 A2 beschrieben.

Weiterhin wurde überraschend festgestellt, dass mit öllöslichen organischen UV-Filtersubstanzen auf Basis von s-Triazinderivaten, die das nachfolgende Strukturmotiv

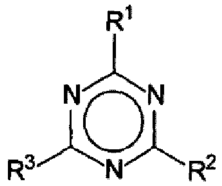

TRIAZIN-GRUND

aufweisen, mit dem obligatorischen O/W-Emulgatorsystem, umfassend mindestens ein Salz von $C_{12-20}$-Alkylphosphat und mindestens ein $C_{14-20}$-Mono- oder Diacylglycerid, teilweise geringere SPF-Werte und geringere Lager- und/oder Photostabilitäten erzielt werden konnten als beispielsweise mit 3,3,5-Trimethylcyclohexylsalicylat. In einer bevorzugten Ausführungsform sind die erfindungsgemäßen Zusammensetzungen daher frei von s-Triazinderivaten, die das oben stehende Strukturmotiv TRIAZIN-GRUND aufweisen.

s-Triazinderivate, die das oben stehende Strukturmotiv TRIAZIN-GRUND aufweisen, sind im Stand der Technik bekannt, beispielsweise aus EP 775698, EP 878469 und EP 1027881.

Hinsichtlich der $C_3$-Achse des Triazin-Grundkörpers dieser Verbindungen sind sowohl symmetrische Substitution als auch unsymmetrische Substitution denkbar.

In diesem Sinne symmetrisch substituierte s-Triazine weisen drei gleiche Substituenten $R^1$, $R^2$ und $R^3$ auf, während unsymmetrisch substituierte s-Triazinderivate demzufolge unterschiedliche Substituenten aufweisen, wodurch die $C_3$-Symmetrie zerstört wird. Im Sinne der vorliegenden Erfindung wird als "unsymmetrisch" stets unsymmetrisch hinsichtlich der $C_3$-Achse des Triazin-grundkörpers verstanden, es sei denn, etwas anderes wäre ausdrücklich erwähnt.

Hinsichtlich der $C_3$-Achse des Triazin-Grundkörpers symmetrische Triazinderivate sind beispielsweise solche der allgemeinen Formel TRIAZIN-GRUND mit $R^1 = R^2 = R^3$ = -NH-Phe-COOR, mit Phe = Phenyl-Rest, bei dem die Substituenten -NH und -COOR in para-Position zueinander stehen. Entsprechende symmetrische Triazinderivate sind 4,4',4"-(1,3,5-Triazin-2,4,6-triyltriimino)-tris-benzoesäure-tris(alkylester), insbesondere 4,4',4"-(1,3,5-Triazin-2,4,6-triyltriimino)-tris-benzoesäure-tris(2-ethylhexylester) [INCI: Ethylhexyl Triazone, vormals Octyl Triazone], das als Einzelsubstanz unter dem Namen UVINUL® T 150 vertrieben wird, aber auch in diversen kommerziellen UV-Filtermischungen erhältlich ist.

Unsymmetrische Triazinderivate sind beispielsweise solche, die in EP 775698 offenbart sind:

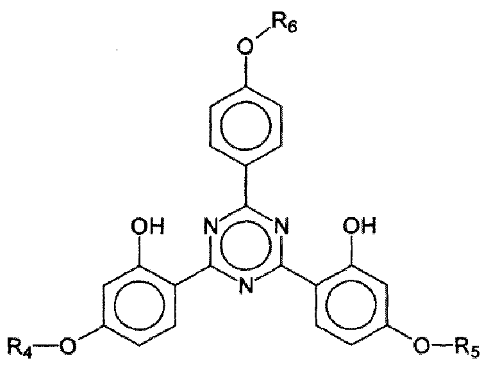

(BIS-RESOR-TRIAZIN)-I

und/oder

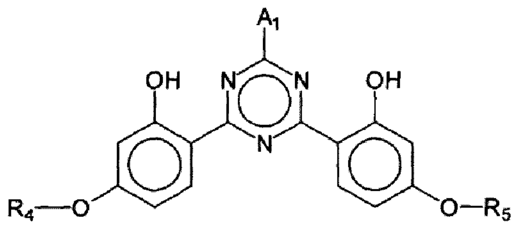

(BIS-RESOR-TRIAZIN)-II

$R_4$ und $R_5$ können aus der Gruppe der verzweigten und unverzweigten Alkylgruppen von 1 bis 18 Kohlenstoffatomen gewählt. Auch können die Alkylgruppen wiederum mit Silyloxygruppen substituiert sein.

$A_1$ stellt beispielsweise einen substituierten homo- oder heterocyclischen aromatischen Fünfring oder Sechsring dar.

In der allgemeinen Formel (BIS-RESOR-TRIAZIN)-I kann $R_6$ ein Wasserstoffatom oder eine verzweigte oder unverzweigte Alkylgruppe mit 1 - 10 Kohlenstoffatomen darstellen. Eine beispielhafte Verbindung der allgemeinen Formel (BIS-RESOR-TRIAZIN)-I, in der $R_4$ und $R_5$ jeweils eine 2-Ethylhexyl-Gruppe und $R_6$ eine Methylgruppe darstellen, ist 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol Methoxyphenyl Triazine), unter dem Handelsnamen Tinosorb® S von CIBA erhältlich.

Eine weitere beispielhafte unsymmetrisch substituierte s-Triazin-Verbindung ist eine Verbindung mit der INCI-Bezeichnung Diethylhexyl Butamido Triazone, mit der allgemeinen Formel TRIAZIN-GRUND mit $R^1$ = $R^2$ -NH-Phe-COOR, mit Phe = Phenyl-Rest, bei dem die Substituenten -NH und -COOR in para-Position zueinander stehen, R = 2-Ethylhexyl und $R^3$ = -NH-Phe-CONH-tert-Butyl, mit Phe = Phenyl-Rest, bei dem die Substituenten -NH und -CONH-tert.-Butyl in para-Position zueinander stehen. Diese UV-Filtersubstanz, ein UV-A-Filter, ist unter der Handelsbezeichnung UVASORB HEB bei Sigma 3V erhältlich ist.

Weitere UV-Filtersubstanzen auf Basis von s-Triazin-Verbindungen sind:

- 2,4,6-Tris([1,1'-Biphenyl]-4-yl)-1,3,5-Triazine, INCI: Tris-Biphenyl Triazine, erhältlich unter dem Handelsnamen Tinosorb A2B von CIBA,
- 2,4-bis-[5-1(di-methylpropyl)benzoxazol-2-yl-(4-phenyl)-imino]-6-(2-ethylhexyl)-imino-1,3,5-triazin (CAS Nr. 288254-16-0, Uvasorb® K2A von 3V Sigma, INCI: Ethylhexyl Bis-Isopentylbenzoxazolylphenyl Melamine),
- 2,4-Bis-{[4-(3-sulfonato)-2-hydroxy-propyloxy)-2-hydroxy]-phenyl}-6-(4-methoxyphenyl)-1,3,5-triazin-Natriumsalz,
- 2,4-Bis-{[4-(3-(2-propyloxy)-2-hydroxy-propyloxy)-2-hydroxy]-phenyl}-6-(4-methoxyphenyl)-1,3,5-triazin,
- 2,4-Bis-{[4-(2-ethyl-hexyloxy)-2-hydroxy]-phenyl}-6-[4-(2-methoxyethyl-carboxyl)-phenylamino]-1,3,5-triazin,
- 2,4-Bis-{[4-(3-(2-propyloxy)-2-hydroxy-propyloxy)-2-hydroxy]-phenyl}-6-[4-(ethylcarboxyl)-phenylamino]-1, 3,5-triazin,
- 2,4-Bis-{[4-(2-ethyl-hexyloxy)-2-hydroxy]-phenyl}-6-(1-methyl-pyrrol-2-yl)-1,3,5-triazin,
- 2,4-Bis-{[4-tris(trimethylsiloxy-silylpropyloxy)-2-hydroxy]-phenyl}-6-(4-methoxyphenyl)-1,3,5-triazin,
- 2,4-Bis-([4-(2-methylpropenyloxy)-2-hydroxy]-phenyl}-6-(4-methoxyphenyl)-1,3,5-triazin und
- 2,4-Bis-{[4-(1',1',1',3',5',5',5'-Heptamethylsiloxy-2-methyl-propyloxy)-2-hydroxy]-phenyl}-6-(4-methoxyphenyl)-1,3,5-triazin,
- 2-[4,6-Bis(2,4-dimethylphenyl)-1,3,5-triazin-2-yl]-5-(ethylhexyloxy)phenol.

Um den Lichtschutzfaktor weiter zu erhöhen, können die erfindungsgemäßen Zusammensetzungen bevorzugt mindestens eine weitere organische und/oder anorganische UV-Filtersubstanz enthalten, die besonders bevorzugt verschieden ist von s-Triazinderivaten, die das oben stehende Strukturmotiv TRIAZIN-GRUND aufweisen, und die außerdem verschieden ist von 2-Ethylhexylsalicylat, von 4-Isopropylbenzylsalicylat und von Polysilicone-15.

Weitere bevorzugte erfindungsgemäße Zusammensetzungen enthalten a) 2-Cyano-3,3-phenylzimtsäure-2-ethylhexylester, b) 4-(tert.-Butyl)-4'-methoxydibenzoylmethan, c) 3,3,5-Trimethylcyclohexylsalicylat und d) 2-Phenylbenzimidazol-5-sulfonsäure-Natriumsalz und sind frei von s-Triazinderivaten, die das oben stehende Strukturmotiv TRIAZIN-GRUND aufweisen.

Weitere bevorzugte erfindungsgemäße Zusammensetzungen enthalten a) 2-Cyano-3,3-phenylzimtsäure-2-ethylhexylester, b) 4-(tert.-Butyl)-4'-methoxydibenzoylmethan, c) 3,3,5-Trimethylcyclohexylsalicylat und d) 2-Phenylbenzimidazol-5-sulfonsäure-Argininsalz und sind frei von s-Triazinderivaten, die das oben stehende Strukturmotiv TRIAZIN-GRUND aufweisen.

Weitere bevorzugte erfindungsgemäße Zusammensetzungen enthalten a) 2-Cyano-3,3-phenylzimtsäure-2-ethylhexylester, b) 4-(tert.-Butyl)-4'-methoxydibenzoylmethan, c) 3,3,5-Trimethylcyclohexylsalicylat und d) Dinatriumphenylbenzimidazoltetrasulfonat und sind frei von s-Triazinderivaten, die das oben stehende Strukturmotiv TRIAZIN-GRUND

aufweisen.

Weitere bevorzugte öllösliche organische UV-Filtersubstanzen, die vorteilhaft in den erfindungsgemäßen Zusammensetzungen eingesetzt werden können, sind im Folgenden genannt.

Benzotriazole

Eine weitere bevorzugte UV-Filtersubstanz ist 2,2'-Methylen-bis-(6-(2H-benzotriazol-2-yl)-4-(1,1,3,3-tetramethylbutyl)-phenol) (INCI: Methylene Bis-Benzotriazolyl Tetramethylbutylphenol, MBBT) mit folgender Strukturformel

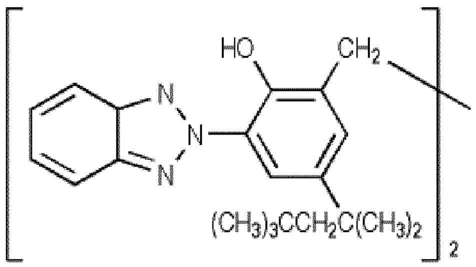

,

unter der Handelsbezeichnung Tinosorb® M von CIBA erhältlich. Hierbei handelt es sich um einen so genannten Breitbandfilter, der sowohl UV-A- als auch UV-B-Strahlung absorbiert.

Eine weitere bevorzugte optionale Breitband-UV-Filtersubstanz ist 2-(2H-Benzotriazol-2-yl)-4-methyl-6-[2-methyl-3-[1,3,3,3-tetramethyl-1-((trimethylsilyl)oxy]disiloxanyl]propyl]-phenol (CAS-Nr.: 155633-54-8) mit der INCI-Bezeichnung Drometrizole Trisiloxane.

Weitere bevorzugte optionale Benzotriazol-Filter sind 2,2'-Methyl-bis-[6(2H-benzotriazol-2-yl)-4-(methyl)phenol] (MIXXIM BB/200 der Firma Fairmount Chemical), 2-(2'-Hydroxy-3',5'-di-t-amylphenyl)benzotriazol (CAS- Nr.: 025973-551), 2-(2'-Hydroxy-5'-octylphenyl)-benzotriazol (CAS-Nr. 003147-75-9), 2-(2'-Hydroxy-5'-methylphenyl)benzotriazol (CAS-Nr. 2440-22-4) und 2-(2H-benzotriazol-2-yl)-4-methyl-6-[2-methyl-3-[1,3,3,3-tetramethyl-1-((trimethylsilyl)oxy]disiloxanyl)propyl]-phenol (CAS-Nr.: 155633-54-8) mit der INCI-Bezeichnung Drometrizole Trisiloxane.

Weitere bevorzugte UV-Filtersubstanzen, die in den erfindungsgemäßen Zusammensetzungen eingesetzt werden können, sind im Folgenden genannt:

- Derivate des Camphers, insbesondere 3-Benzylidencampher-Derivate, die keine ionisierbaren funktionellen Gruppen im Molekül aufweisen können, bevorzugt 3-(4'-Methylbenzyliden)-D,L-campher [INCI: 4-Methylbenzylidene Camphor];
- Derivate des Camphers, die ionisierbare funktionelle Gruppen im Molekül aufweisen können, bevorzugt Sulfonsäure-Derivate des 3-Benzylidencamphers, wie z. B. 4-(2-Oxo-3-bornylidenmethyl)benzolsulfonsäure, 2-Methyl-5-(2-oxo-3-bornylidenmethyl)sulfonsäure und deren Salze; das 1,4-di(2-oxo-10-Sulfo-3-bornylidenmethyl)-Benzol und dessen Salze (besonders die entsprechenden 10-Sulfato-verbindungen, insbesondere das entsprechende Natrium-, Kalium- oder Triethanolamin-Salz), das auch als Benzol-1,4-di(2-oxo-3-bornylidenmethyl-10-sulfonsäure) bezeichnet wird, Sulfonsäurederivate des 3-Benzylidencamphers, wie z. B. 4-(2-Oxo-3-bornylidenmethyl)benzolsulfonsäure und 2-Methyl-5-(2-oxo-3-bornyliden)sulfonsäure und deren Salze mit der INCI-Bezeichnung Terephthalylidene Dicamphor Sulfonic Acid (CAS.-Nr.: 92761-26-7, als Mexoryl SX von der Firma Chimex erhältlich);
- 4-Aminobenzoësäure-Derivate, bevorzugt 4-(Dimethylamino)-benzoesäure(2-ethylhexyl)ester, 4-(Dimethylamino)benzoesäureamylester;
- 2-Aminobenzoësäure-Derivate
- 4-Methoxyzimtsäure(2-ethylhexyl)ester,
- 4-Methoxyzimtsäurepropylester,
- 4-Methoxyzimtsäureisopentylester;
- Derivate des Benzophenons, die keine ionisierbaren funktionellen Gruppen im Molekül aufweisen, bevorzugt 2-Hydroxy-4-methoxy-4'-methylbenzophenon, 2,2'-Dihydroxy-4-methoxybenzophenon, 2-Hydroxy-4-methoxybenzophenon, 2-Hydroxy-4-methoxy-4'-methylbenzophenon, 2,2'-Dihydroxy-4-methoxybenzophenon; 2-(4'-Diethylamino-2'-hydroxybenzoyl)-benzoesäurehexylester (auch: Aminobenzophenon, unter der Bezeichnung Uvinul A Plus von BASF erhältlich),
- Derivate des Benzophenons, die ionisierbare funktionelle Gruppen im Molekül aufweisen, bevorzugt Sulfonsäurederivate von Benzophenonen, besonders bevorzugt 2-Hydroxy-4-methoxybenzophenon-5-sulfonsäure und ihre Salze,
- Ester der Benzalmalonsäure, bevorzugt 4-Methoxybenzalmalonsäuredi(2-ethylhexyl)ester,

- an Polymere gebundene UV-Filter, die von Polysilicone-15 verschieden sind;
- Derivate von Benzoxazol, bevorzugt 2,2'(Naphthalene-1,4-Diyl)bis(benzoxazole) (INCI: Dibenzoxazoyl Naphthalene) und 2,4-bis-[5-1(di-methylpropyl)benzoxazol-2-yl-(4-phenyl)-imino]-6-(2-ethylhexyl)-imino-1,3,5-triazin (Uvasorb® K2A von 3V Sigma, INCI: Ethylhexyl Bis-Isopentylbenzoxazolylphenyl Melamine). Die Benzoxazol-Derivate liegen bevorzugt in gelöster Form in den erfindungsgemäßen kosmetischen Zusammensetzungen vor. Es kann ggf. aber auch von Vorteil sein, wenn die Benzoxazol-Derivate in pigmentärer, d. h. ungelöster Form - beispielsweise in Partikelgrößen von 10 nm bis zu 300 nm - vorliegen.

Bevorzugte erfindungsgemäße Zusammensetzungen enthalten, jeweils bezogen auf ihr Gesamtgewicht, mindestens einen organischen UV-Filter in ungelöster Form in einer Gesamtmenge von 0,1 - 6 Gew.-%, bevorzugt 0,5 - 5 Gew.-% und besonders bevorzugt 1 - 3 Gew.-%.
Einige der öllöslichen UV-Filter können selbst als Lösungsmittel oder Lösungsvermittler für andere UV-Filter dienen. So lassen sich beispielsweise Lösungen des UV-A-Filters 1-(4-tert.-Butylphenyl)-3-(4'-methoxyphenyl)propan-1,3-dion in verschiedenen UV-B-Filtern herstellen. Die erfindungsgemäßen Zusammensetzungen enthalten daher in einer weiteren bevorzugten Ausführungsform 1-(4-tert.-Butylphenyl)-3-(4'-methoxyphenyl)propan-1,3-dion in Kombination mit mindestens einem UV-B-Filter, ausgewählt aus 4-Methoxyzimtsäure-2-ethylhexylester, 2-Cyano-3,3-phenylzimtsäure-2-ethylhexylester, Salicylsäure-2-ethylhexylester und 3,3,5-Trimethyl-cyclohexylsalicylat. In diesen Kombinationen liegt das Gewichtsverhältnis von UV-B-Filter zu 1-(4-tert.-Butylphenyl)-3-(4'-methoxyphenyl)propan-1,3-dion zwischen 1:1 und 10:1, bevorzugt zwischen 2:1 und 8:1, das molare Verhältnis liegt entsprechend zwischen 0,3 und 3,8, bevorzugt zwischen 0,7 und 3,0, besonders bevorzugt bei 0,5 - 0,75, außerordentlich bevorzugt bei 0,6 - 0,9.
In weiteren bevorzugten Kombinationen liegt das Gewichtsverhältnis von UV-B-Filter zu 1-(4-tert.-Butylphenyl)-3-(4'methoxyphenyl)propan-1,3-dion im Bereich von 0,5 bis 6, bevorzugt im Bereich von 0,6 bis 3, das molare Verhältnis liegt entsprechend im Bereich von 0,3 bis 3, bevorzugt im Bereich von 0,5 bis 1,5, besonders bevorzugt bei 0,5 - 0,75, außerordentlich bevorzugt bei 0,6 - 0,9. In weiteren bevorzugten Kombinationen liegt das Gewichtsverhältnis von Octocrylene zu 1-(4-tert.-Butylphenyl)-3-(4'methoxyphenyl)propan-1,3-dion im Bereich von 0,5 bis 6, bevorzugt im Bereich von 0,6 bis 3, das molare Verhältnis liegt entsprechend im Bereich von 0,3 bis 3, bevorzugt im Bereich von 0,5 bis 1,5, besonders bevorzugt bei 0,5 - 0,75, außerordentlich bevorzugt bei 0,6 -0,9.
Bei den erfindungsgemäß bevorzugten optionalen anorganischen UV-Filtersubstanzen handelt es sich bevorzugt um feindisperse oder kolloiddisperse Metalloxide und Metallsalze, beispielsweise Titandioxid, Zinkoxid, Eisenoxid, Aluminiumoxid, Ceroxid, Zirkoniumoxid, Silicate (Talk) und Bariumsulfat. Besonders bevorzugt sind Titandioxid und Zinkoxid. Die Partikel sollten dabei einen zahlenmittleren Partikeldurchmesser von weniger als 100 nm, bevorzugt zwischen 5 und 50 nm und insbesondere zwischen 15 und 30 nm aufweisen, also so genannte Nanopigmente darstellen. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z. B. Titandioxid T 805 (Degussa) oder Eusolex® T2000 (Merck). Als hydrophobe Coatingmittel kommen dabei vor allem Silicone und dabei speziell Trialkoxyoctylsilane, bevorzugt Triethoxy Caprylylsilane, oder Simethicone in Frage. Weitere bevorzugte Coatingmittel sind Aluminiumoxide. Weitere bevorzugte Coatingmittel ist Siliciumdioxid, z. B. bei dem Handelsprodukt Eusolex T AVO von Merck KGaA. Ein weiteres bevorzugtes Coatingmittel ist Stearinsäure. Ein weiteres bevorzugtes Coatingmittel ist Polyhydroxystearinsäure. Ein weiteres bevorzugtes Coatingmittel ist Aluminiumstearat. Ein besonders bevorzugter anorganischer UV-Filter ist ein mit Triethoxy Caprylylsilane hydrophob beschichtetes Titandioxid, erhältlich unter der Bezeichnung Titan M 265 von Kemira. Erfindungsgemäß bevorzugt ist mindestens eine anorganische UV-Filtersubstanz in einer Gesamtmenge von 0,1 - 10 Gew.-%, besonders bevorzugt 0,5 - 7 Gew.-%, außerordentlich bevorzugt 1,0 - 5 Gew.-% und weiter bevorzugt 2,0 - 4,0 Gew.-%, jeweils bezogen auf die gesamte Zusammensetzung, enthalten.
Um die haptischen Eigenschaften und/oder den Lichtschutzfaktor der erfindungsgemäßen Zusammensetzungen weiter zu verbessern, können bevorzugte erfindungsgemäße Zusammensetzungen unter Normalbedingungen feste, ungelöste Partikel mit einem zahlenmittleren Durchmesser von mindestens 350 nm, die in der Zusammensetzung unlöslich sind, enthalten. Besonders bevorzugt weisen derartige feste, ungelöste Partikel einen zahlenmittleren Durchmesser von 500 nm bis 80 μm, außerordentlich bevorzugt von 1 μm bis 20 μm, auf. Bevorzugte feste, ungelöste Partikel mit einem zahlenmittleren Durchmesser von mindestens 350 nm, die in der Zusammensetzung unlöslich sind, sind sphärische Silica-Partikel. Besonders bevorzugt sind sphärische Silica-Partikel, die nicht organisch oder anorganisch modifiziert sind und keine organische oder anorganische Beschichtung aufweisen. Weiterhin bevorzugt sind sphärische Silica-Partikel, die einen zahlenmittleren Partikeldurchmesser im Bereich von 2 - 15 μm, bevorzugt 5 - 10 μm, aufweisen. Ein bevorzugtes Handelsprodukt mit derartigen sphärischen, nicht organisch oder anorganisch modifizierten, nicht organisch oder anorganisch beschichteten Silica-Partikeln ist Cosmedia Silc von BASF/ Cognis. Weiterhin bevorzugt sind feste, ungelöste Partikel mit einem zahlenmittleren Durchmesser von mindestens 350 nm, die in der Zusammensetzung unlöslich sind, ausgewählt aus METHYL METHACRYLATE CROSSPOLYMER (PMMA). Besonders bevorzugte PMMA-Partikel sind die Handelsprodukte SUNPMMA-S und SUNSIL Tin 30 von Sunjin Chemicals Co. mit einem zahlenmittleren Partikeldurchmesser von 5 - 10 μm bzw. 2 - 7 μm. Besonders bevorzugt sind feste, ungelöste Partikel, die in der Zusammensetzung unlöslich sind, in einer Gesamtmenge von 0 bis 3 Gew.-%, bezogen auf das Gesamtgewicht der Zusammensetzung.

Um die kosmetischen Eigenschaften der erfindungsgemäßen Zusammensetzungen weiter zu verbessern, können die erfindungsgemäßen Zusammensetzungen bevorzugt feste, ungelöste Partikel, die in der Zusammensetzung unlöslich sind, ausgewählt aus färbenden, farbgebenden, mattierenden oder glanzgebenden Pigmenten, enthalten. Bevorzugte Pigmente dieser Art können anorganisch oder organisch sein. Weitere bevorzugte Pigmente weisen einen zahlenmittleren Teilchendurchmesser von 0,1 - 200 μm, bevorzugt 0,5 - 100 μm, besonders bevorzugt 1 - 50 μm und außerordentlich bevorzugt 2 - 30 μm auf. Besonders bevorzugte anorganische Pigmente sind ausgewählt aus den Oxiden von Silicium, Titan, Eisen, Zink, Zirkonium, Magnesium, Cer und Bismut, aus Bismutoxychlorid, Bornitrid, Glimmer, Flussspat und wasserunlöslichen Perlglanzpigmenten, die mit mindestens einer anorganischen und/oder organischen Verbindung beschichtet sein können. Weitere bevorzugte Pigmente sind ausgewählt aus farbigen und farblosen Pigmenten. Einige der im Folgenden genannten Pigmente dienen auch als UV-Absorber. Besonders bevorzugte farbige Pigmente sind ausgewählt aus den Eisenoxiden mit den Color Index-Nummern CI 77491 (Eisenoxid rot), CI 77492 (Eisenoxidhydrat gelb) und CI 77499 (Eisenoxid schwarz), aus CI 77891 (Titandioxid) und Ruß.

Besonders bevorzugte optionale anorganische Pigmente sind beschichtet. Die Beschichtung kann mit Hilfe von anorganischen und/oder organischen Verbindungen erfolgen. Erfindungsgemäß besonders bevorzugt sind anorganische Pigmente, die eine anorganische Beschichtung aufweisen. Außerordentlich bevorzugte Pigmente dieser Art sind ausgewählt aus Siliciumdioxid-Partikeln, die mit Titandioxid und/oder Eisenoxiden beschichtet sind. Ein besonders bevorzugtes Pigment dieser Art ist das Handelsprodukt Ronasphere® LDP der Firma Merck KGaA. Bei diesem Produkt handelt es sich um sphärische Siliciumdioxid-Partikel, die mit Titandioxid und Eisenoxid beschichtet sind. Ronasphere® LDP weist einen zahlenmittleren Teilchendurchmesser von 4 - 7 μm auf.

Weiterhin bevorzugt sind anorganisch beschichtete Glimmerpigmente, die keinen Perlglanz aufweisen. Weitere bevorzugte optionale anorganisch beschichtete anorganische Pigmente sind Glimmerpigmente, die mit Titandioxid in verschiedenen Schichtdicken beschichtet sind, beispielsweise die Produkte der Timiron®-Serie von Rona/Merck KGaA, insbesondere Pigmente der Produktreihen Timiron® MP, Timiron® Super, Timiron® Starlight und Timiron® Silk. Die genannten Produkte weisen mittlere Teilchendurchmesser von 5 - 60 μm bzw. 10 - 60 μm bzw. 10 - 125 μm bzw. 5 - 25 μm auf. Ebenfalls bevorzugt sind Glimmerpartikel mit einer Beschichtung aus Titandioxid und Eisenoxid, z. B. die Handelsprodukte Timiron® MP-20, MP-24, MP-25, MP-28, MP-29, MP-60 und MP-65. Weiterhin erfindungsgemäß bevorzugt sind mit Titandioxid und/oder rotem und/oder schwarzem Eisenoxid beschichtete Glimmerpartikel, z. B. die Produkte der Colorona®-Serie. Weitere bevorzugte Pigmente sind mit Kieselgel beschichtete Glimmerpigmente, z. B. das Handelsprodukt Micronasphere® M. Weitere erfindungsgemäß bevorzugte Pigmente sind anorganisch beschichtete anorganische Pigmente, deren Beschichtung einen Anteil von 0,1 - 1 Gew.-% Zinnoxid aufweist. Ebenfalls erfindungsgemäß bevorzugt sind anorganische Pigmente, die mit organischen Substanzen beschichtet sind. Bevorzugte Beispiele hierfür sind mit Aluminiumstearat beschichtete Titandioxid-Pigmente (z. B. das Handelsprodukt MT 100 T von der Firma Tayca), mit Dimethylpolysiloxan (Dimethicone) beschichtetes Zinkoxid, mit Dimethicone beschichtetes Bornitrid (Tres BN® UHP 1106 von Carborundum), mit einem Gemisch aus Dimethylpolysiloxan und Silicagel (Simethicone) und Aluminiumoxidhydrat (Alumina) beschichtetes Titandioxid (Eusolex® T 2000 von Merck), mit Octylsilanol beschichtetes Titandioxid oder sphärische Polyalkylsesquisiloxan-Partikel (z. B. Aerosil® R972 von Evonik). Weitere bevorzugte feste, ungelöste Partikel, die in der Zusammensetzung unlöslich sind, sind die durch Hydrolyse und Kondensationsreaktionen von Methyltrimethoxysilane erhältlichen Polymethylsilsesquioxane, die sphärisch sein können (insbesondere Aerosil® R 972 und Aerosil® 200 V von Evonik Degussa), und deren Teilchengrößenverteilung durch die Herstellung gesteuert werden kann.

Die festen, ungelösten Partikel können sowohl einzeln als auch im Gemisch eingesetzt werden.

Die Gesamtmenge der festen, ungelösten Partikel, die in der Zusammensetzung unlöslich sind, beträgt bevorzugt 0,1 - 5 Gew.-%, besonders bevorzugt 0,5 - 3 Gew.-%, außerordentlich bevorzugt 1,0 - 2,0 Gew.-%, jeweils bezogen auf das Gesamtgewicht der erfindungsgemäßen Zusammensetzung.

Die erfindungsgemäßen Zusammensetzungen können ferner kosmetische Hilfsstoffe und weitere Wirkstoffe enthalten, wie sie üblicherweise in solchen Zusammensetzungen verwendet werden, z. B. Konservierungsmittel, Konservierungshelfer, Bakterizide, Parfüms, Farbstoffe, anfeuchtende und/oder feuchthaltende Substanzen, Substanzen zur Einstellung des pH-Wertes, Komplexbildner wie EDTA, NTA, β-Alanindiessigsäure und Phosphonsäuren, Treibmittel wie Propan-Butan-Gemische, Pentan, Isopentan, Isobutan, $N_2O$, Dimethylether, $CO_2$ und Luft, Vitamine oder Antifalten-Wirkstoffe.

Vorteilhafte Konservierungsmittel sind beispielsweise Iodopropylbutylcarbamate, Parabene, Phenoxyethanol, Ethanol, Benzoesäure und andere. Bevorzugt umfasst das Konservierungssystem ferner auch Konservierungshelfer, wie beispielsweise Octoxyglycerin, 1,2-Pentandiol, 1,5-Pentandiol, 1,2-Hexandiol, 1,6-Hexandiol, 1,2-Heptandiol, 1,2-Octandiol, 1,8-Octandiol, 1,2-Nonandiol, 1,2-Decandiol, 1,10-Decandiol, 1,2-Undecandiol, 1,2-Dodecandiol, 1,2-Tridecandiol oder 1,2-Tetradecandiol.

Besonders bevorzugte Zusammensetzungen werden ferner erhalten, wenn als Zusatz- oder Wirkstoffe Antioxidantien eingesetzt werden. Erfindungsgemäß bevorzugt enthalten die Zusammensetzungen mindestens ein Antioxidans. Als günstige, aber dennoch fakultativ zu verwendende Antioxidantien können alle für kosmetische und/oder dermatologische Anwendungen geeigneten oder gebräuchlichen Antioxidantien verwendet werden, insbesondere Tocopherol, Tocopherylacetat oder Dimethylmethoxy Chromanol, erhältlich unter dem Handelsnamen Lipochroman-6 von Lipotec.

Weiter vorteilhaft werden der oder die Wirkstoffe gewählt aus der Gruppe, die Catechine und Gallensäureester von Catechinen und wässrige bzw. organische Extrakte aus Pflanzen oder Pflanzenteilen umfasst, die einen Gehalt an Catechinen oder Gallensäureestern von Catechinen aufweisen, wie beispielsweise den Blättern der Pflanzenfamilie Theaceae, insbesondere der Spezies Camellia sinensis (grüner Tee). Insbesondere vorteilhaft sind deren typische Inhaltsstoffe (z. B. Polyphenole bzw. Catechine, Coffein, Vitamine, Zucker, Mineralien, Aminosäuren, Lipide). Catechine stellen eine Gruppe von Verbindungen dar, die als hydrierte Flavone oder Anthocyanidine aufzufassen sind und Derivate des "Catechins" (Catechol, 3,3',4',5,7-Flavanpentaol, 2-(3,4-Dihydroxyphenyl)-chroman-3,5,7-triol) darstellen. Auch Epicatechin ((2R, 3R)-3,3',4',5,7-Flavanpentaol) ist ein vorteilhafter Wirkstoff im Sinne der vorliegenden Erfindung.

In einer weiteren bevorzugten Ausführungsform enthalten die erfindungsgemäßen Zusammensetzungen mindestens ein Flavonoid oder mindestens einen Flavonoid-reichen Pflanzenextrakt. Die erfindungsgemäß bevorzugten Flavonoide umfassen die Glycoside der Flavone, der Flavanone, der 3-Hydroxyflavone (Flavonole), der Aurone und der Isoflavone. Besonders bevorzugte Flavonoide sind ausgewählt aus Naringin (Aurantiin, Naringenin-7-rhamnoglucosid), $\alpha$-Glucosylrutin, $\alpha$-Glucosylmyricetin, $\alpha$-Glucosylisoquercetin, $\alpha$-Glucosylquercetin, Dihydroquercetin (Taxifolin), Hesperidin (3',5,7-Trihydroxy-4'-methoxyflavanon-7-rhamnoglucosid, Hesperitin-7-O-rhamnoglucosid), Neohesperidin, Rutin (3,3',4',5,7-Pentahydroxyflavon-3-rhamnoglucosid, Quercetin-3-rhamnoglucosid), Troxerutin (3,5-Dihydroxy-3',4',7-tris(2-hydroxyethoxy)-flavon-3-(6-O-(6-deoxy-$\alpha$-L-mannopyranosyl)-$\beta$-D-glucopyranosid)), Monoxerutin (3,3',4',5-Tetrahydroxy-7-(2-hydroxyethoxy)-flavon-3-(6-O-(6-deoxy-$\alpha$-L-mannopyranosyl)-$\beta$-D-glucopyranosid)), Diosmin (3',4',7-Trihydroxy-5-methoxyflavanon-7-rhamnoglucosid), Eriodictin und Apigenin-7-glucosid (4',5,7-Trihydroxyflavon-7-glucosid). Erfindungsgemäß außerordentlich bevorzugte Flavonoide sind $\alpha$-Glucosylrutin, Naringin und Apigenin-7-glucosid.

Ebenfalls bevorzugt sind die aus zwei Flavonoideinheiten aufgebauten Biflavonoide, die z. B. in Gingko-Arten vorkommen. Weitere bevorzugte Flavonoide sind die Chalkone, vor allem Phloricin, Hesperidinmethylchalkon und Neohesperidindihydrochalkon.

Erfindungsgemäß besonders bevorzugte Zusammensetzungen enthalten mindestens ein Flavonoid in einer Gesamtmenge von 0,0001 bis 1 Gew.-%, bevorzugt 0,0005 bis 0,5 Gew.-% und besonders bevorzugt 0,001 bis 0,1 Gew.-%, jeweils bezogen auf die Flavonoidaktivsubstanz in der gesamten kosmetischen Zusammensetzung.

In einer weiteren bevorzugten Ausführungsform enthalten die erfindungsgemäßen Zusammensetzungen mindestens ein Ubichinon oder ein Ubichinol oder deren Derivate. Ubichinole sind die reduzierte Form der Ubichinone. Die erfindungsgemäß bevorzugten Ubichinone weisen die Formel (UBI-I) auf:

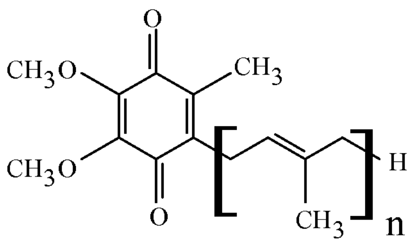

(UBI-I)

mit n = 6, 7, 8, 9 oder 10.

Besonders bevorzugt ist das Ubichinon der Formel (UBI-I) mit n = 10, auch bekannt als Coenzym Q10.

Erfindungsgemäß besonders bevorzugte Zusammensetzungen enthalten mindestens ein Ubichinon, Ubichinol oder ein Derivat hiervon in einer Gesamtmenge von 0,0001 - 1 Gew.-%, bevorzugt 0,001 - 0,5 Gew.-% und besonders bevorzugt 0,005 - 0,1 Gew.-%, jeweils bezogen auf die gesamte Zusammensetzung.

In einer weiteren bevorzugten Ausführungsform enthalten die erfindungsgemäßen Zusammensetzungen mindestens ein natürlich vorkommendes Xanthin-Derivat, ausgewählt aus Coffein, Theophyllin, Theobromin und Aminophyllin. Erfindungsgemäß bevorzugt sind die natürlich vorkommenden Xanthin-Derivate in Mengen von 0,0001 bis 1 Gew.-%, besonders bevorzugt 0,001 bis 0,5 Gew.-% und außerordentlich bevorzugt 0,005 bis 0,1 Gew.-%, jeweils bezogen auf die gesamte Zusammensetzung, enthalten.

In einer weiteren bevorzugten Ausführungsform enthalten die erfindungsgemäßen Zusammensetzungen Ectoin. Ectoin ist der Trivialname für 2-Methyl-1,4,5,6-tetrahydropyrimidin-4-carboxylat. Erfindungsgemäß bevorzugt ist Ectoin in Mengen von 0,0001 bis 1 Gew.-%, besonders bevorzugt 0,001 bis 0,5 Gew.-% und außerordentlich bevorzugt 0,005 bis 0,01 Gew.-%, jeweils bezogen auf die gesamte Zusammensetzung, enthalten.

In einer weiteren bevorzugten Ausführungsform enthalten die erfindungsgemäßen Zusammensetzungen mindestens eine natürliche Betainverbindung. Die Herkunft der eingesetzten natürlichen Betainverbindung kann erfindungsgemäß durchaus synthetisch sein. Erfindungsgemäß bevorzugte natürliche Betainverbindungen sind natürlich vorkommende Verbindungen mit der Atomgruppierung $R_3N^+\text{-}CH_2\text{-}X\text{-}COO^-$ gemäß IUPAC-Regel C-816.1. Sogenannte Betaintenside (synthetisch) fallen nicht unter die erfindungsgemäß verwendeten Betainverbindungen, ebenso wenig andere zwitterionische Verbindungen, in denen sich die positive Ladung an N oder P und die negative Ladung formal an O, S, B oder C befindet, die aber nicht der IUPAC-Regel C-816.1 entsprechen. Erfindungsgemäß bevorzugte Betainverbindungen sind Betain ($Me_3N^+\text{-}CH_2\text{-}COO^-$), Carnitin ($Me_3N^+\text{-}CH_2\text{-}CHOH\text{-}CH_2\text{-}COO^-$), jeweils mit Me = Methyl, und Ergothionein = 2-Mercapto-$N^\alpha N^\alpha N^\alpha$-trimethyl-L-histidinium-Betain der Formel (BETA-II)

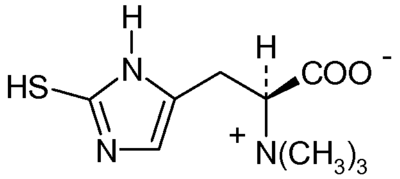

(BETA-II).

Weiterhin können auch Salze und/oder Ester der natürlichen Betainverbindungen erfindungsgemäß bevorzugt eingesetzt werden. Besonders bevorzugte derartige Derivate sind Carnitintartrat, Propionylcarnitin und insbesondere Acetylcarnitin. Beide Enantiomere (D-und L-Form) sind vorteilhaft im Sinne der vorliegenden Erfindung zu verwenden. Es kann auch von Vorteil sein, beliebige Enantiomerengemische, beispielsweise ein Racemat aus D-und L-Form, zu verwenden. Bevorzugte erfindungsgemäße Zusammensetzungen sind dadurch gekennzeichnet, dass sie mindestens eine natürliche Betainverbindung in einer Gesamtmenge von 0,01 bis 5 Gew.-%, bevorzugt 0,1 bis 3 Gew.-%, besonders bevorzugt 0,2 bis 1 Gew.-% und außerordentlich bevorzugt 0,3 - 0,5 Gew.-%, jeweils bezogen auf die gesamte erfindungsgemäße Zusammensetzung, enthalten.

Die erfindungsgemäßen kosmetischen oder dermatologischen Zusammensetzungen liegen in Form einer flüssigen, fließfähigen oder festen Öl-in-Wasser-Emulsion vor.

Weitere bevorzugte Zusatzstoffe sind Verdickungsmittel, z. B. anionische Polymere aus Acrylsäure, Methacrylsäure, Crotonsäure, Maleinsäureanhydrid und 2-Acrylamido-2-methylpropansulfonsäure, wobei die sauren Gruppen ganz oder teilweise als Natrium-, Kalium-, Ammonium-, Mono- oder Triethanolamin-Salz vorliegen können und wobei mindestens ein nichtionisches Monomer enthalten sein kann. Bevorzugte nichtionogene Monomere sind Acrylamid, Methacrylamid, Acrylsäureester, Methacrylsäureester, Vinylpyrrolidon, Vinylether und Vinylester. Bevorzugte anionische Copolymere sind Acrylsäure-Acrylamid-Copolymere sowie insbesondere Polyacrylamidcopolymere mit Sulfonsäuregruppen-haltigen Monomeren, wobei Acryloyldimethyltaurat ein besonders bevorzugtes Sulfonsäuregruppen-haltiges Monomer darstellt. Diese Copolymere können bevorzugt vernetzt vorliegen. Geeignete Handelsprodukte sind Sepigel®305, Simulgel® 600, Simulgel® NS, Simulgel® EPG und Simulgel® EG von SEPPIC. Weitere besonders bevorzugte anionische Homo- und Copolymere sind unvernetzte und vernetzte Polyacrylsäuren. Solche Verbindungen sind zum Beispiel die Handelsprodukte Carbopol®. Ein besonders bevorzugtes anionisches Copolymer enthält als Monomer zu 80 - 98 % eine ungesättigte, gewünschtenfalls substituierte $C_{3-6}$-Carbonsäure oder ihr Anhydrid sowie zu 2 - 20 % gewünschtenfalls substituierte Acrylsäureester von gesättigten $C_{10-30}$-Carbonsäuren, wobei das Copolymer mit den vorgenannten Vernetzungsagentien vernetzt sein kann. Entsprechende Handelsprodukte sind Pemulen® und die Carbopol®-Typen 954, 980, 1342 und ETD 2020 (ex Noveon/Lubrizol).

Geeignete nichtionische Polymere sind beispielsweise Polyvinylalkohole, die teilverseift sein können, z. B. die Handelsprodukte Mowiol® sowie Vinylpyrrolidon/Vinylester-Copolymere und Polyvinylpyrrolidone, die z. B. unter dem Warenzeichen Luviskol® (BASF) vertrieben werden.

Bevorzugte erfindungsgemäße Sonnenschutz-Zusammensetzungen enthalten zur Verdickung der wässrigen Phase und damit zur zusätzlichen Stabilisierung der gesamten Zusammensetzung eine Mischung aus Xanthan-Gum, mindestens einem vernetzten Polyacrylat und mindestens einem vernetzten Acryloyldimethyltaurat-Copolymer. Besonders bevorzugt sind Mischungen aus Xanthan-Gum, mindestens einem vernetzten Polyacrylat und mindestens einem vernetzten Acryloyldimethyltaurat-Copolymer in einer Gesamtmenge von 0,5 - 1,5 Gew.-%, bevorzugt 0,7 - 1,3 Gew.-% und außerordentlich bevorzugt 1 - 1,2 Gew.-%, jeweils bezogen auf das Gesamtgewicht der Zusammensetzung.

In einer weiteren bevorzugten Ausführungsform liegen die erfindungsgemäßen Zusammensetzungen in einem Sprühspender oder Pumpspender verpackt vor.

Gegenstand der Erfindung ist ferner die Verwendung der erfindungsgemäßen vorstehend definierten Zusammensetzungen zum Schutz von Haut und Haar gegen die schädlichen Wirkungen von UV-Strahlung, insbesondere UV-A-und UV-B-Strahlung, insbesondere Sonnenstrahlung.

Ein weiterer Gegenstand der vorliegenden Anmeldung ist die kosmetische Verwendung der vorstehend definierten erfindungsgemäßen oder erfindungsgemäß bevorzugten Zusammensetzungen, insbesondere einer Zusammensetzung gemäß einem der Ansprüche 1, 2, 3, 4, 5, 6, 7, 8, 9, 10, 11, 12, 13 oder 14 zur Verzögerung der Lichtalterung der Haut und/oder zur kontrollierten Bräunung der Haut.

Bezüglich weiterer bevorzugter Ausführungsformen der erfindungsgemäßen Verwendungen gilt mutatis mutandis das zu den erfindungsgemäßen Zusammensetzungen Gesagte.

Ein weiterer Gegenstand der vorliegenden Anmeldung ist ein nicht-therapeutisches, kosmetisches Verfahren zum Schutz von Haut und Haar gegen die schädlichen Wirkungen von UV-Strahlung, insbesondere UV-A-und UV-B-Strahlung, insbesondere Sonnenstrahlung, bei dem eine erfindungsgemäße oder erfindungsgemäß bevorzugte Zusammensetzung, insbesondere eine Zusammensetzung gemäß einem der Ansprüche 1, 2, 3, 4, 5, 6, 7, 8, 9, 10, 11, 12, 13 oder 14 in einer wirksamen Menge auf die Haut aufgetragen wird.

Ein weiterer Gegenstand der vorliegenden Anmeldung ist ein kosmetisches Verfahren zur Verzögerung der Lichtalterung der Haut und/oder zur kontrollierten Bräunung der Haut, bei dem eine erfindungsgemäße oder erfindungsgemäß bevorzugte Zusammensetzung, insbesondere eine Zusammensetzung gemäß einem der Ansprüche 1, 2, 3, 4, 5, 6, 7, 8, 9, 10, 11, 12, 13 oder 14 in einer wirksamen Menge auf die Haut aufgetragen wird.

Bezüglich weiterer bevorzugter Ausführungsformen des erfindungsgemäßen Verfahrens gilt mutatis mutandis das zu den erfindungsgemäßen Zusammensetzungen Gesagte.

Die nachfolgenden Beispiele sollen den Gegenstand der vorliegenden Anmeldung verdeutlichen, ohne ihn hierauf einzuschränken. Die Zahlenwerte in den Beispielen bedeuten Gewichtsprozente, bezogen auf das Gesamtgewicht der jeweiligen Zusammensetzungen.

Liste der verwendeten Rohstoffe

| Handelsname | INCI | Hersteller |
|---|---|---|
| AMP-ULTRA PC 2000 | Aminomethyl propanol (2-Amino-2-methylpropanol), 95 Gew.-%(und 5 Gew.-% Wasser) | Dow (Angus Chemie) |
| Aristoflex AVC | AMMONIUM ACRYLOYLDIMETHYLTAURATE/VP COPOLYMER, T-BUTYL ALCOHOL | Clariant |
| Carbopol ETD 2020 | Acrylates/C10-30 Alkyl Acrylate Crosspolymer | Lubrizol |
| Cetiol B | DIBUTYL ADIPATE | Cognis |
| Controx KS | Tocopherol, Hydrogenated Palm Glycerides Citrate | Cognis |
| Cosmedia Silc | Silica (spherical) | Cognis |
| Cosmedia SP | Sodium Polyacrylate | Cognis |
| Emulsiphos 677660 | Potassium Cetyl Phosphate, Hydrogenated Palm Glycerides | Symrise |
| Lanette 22 | Behenyl alcohol (C22-Alkohol: 70-80 Gew.-%, C20-Alkohol: 10-20 Gew.-%, C18-Alkohol: 5-15 Gew.-%, C16-Alkohol: 0-0,5 Gew.-%, C24-Alkohol: 0-1 Gew.-%) | BASF/ Cognis |
| RonaCare AP | Bis-Ethylhexyl Hydroxydimethoxy Benzylmalonate | Merck KGaA |
| Myritol 318 | Caprylic/Capric Triglyceride | BASF/ Cognis |
| Neo Heliopan AP | Disodium Phenyl Dibenzimidazole Tetrasulfonate | Symrise |
| Neo Heliopan Hydro | Phenylbenzimidazol sulfonic acid | Symrise |
| Olivem 1000 | Cetearyl Olivate, Sorbitan Olivate | Erbslöh |
| PCL Solid 660086 | STEARYLHEPTANOAT + STEARYLOCTANOAT (STEARYL HEPTANOATE, STEARYL CAPRYLATE) | Symrise |
| SymMollient S | n-Hexadecyl-n-nonanoat + n-Octadecyl-n-nonanoat (Cetearyl Nonanoate) | Symrise |

Ergebnisse: Öl-in-Wasser-Emulsionen mit Lichtschutzfaktor (Mengenangaben in Gew.-%)

| | Creme A (erfindungsgemäß | Creme B (V) | Creme C (V) | Creme D (V) | Creme E (V) |
|---|---|---|---|---|---|
| Mischung aus Dikaliummonocetylphosphat und Kaliumdicetylphosphat (ex Emulsiphos 677660) | 1,5 | 1,8 | 1,8 | 1,2 | - |
| Hydrogenated Palm Glycerides (ex Emulsiphos 677660) | 0,9 | 1,2 | 1,2 | 0,8 | - |
| Lanette 22 | 1 | 1,5 | 3 | 3 | 3 |
| Cetearyl Alcohol | - | 0,5 | - | - | - |
| SymMollient S | 1 | - | - | - | - |
| PCL Solid 660086 | - | 1 | - | - | - |
| Bienenwachs | - | - | 0,2 | 0,2 | 0,2 |
| 2-Ethylhexyl-3,5,5-trimethylhexanoat | 3,5 | - | - | - | - |
| Octansäure-4-methyl-2-pentylbutylester | 2 | 2 | 1 | 1 | 1 |
| Cetiol B | - | - | 5 | 5 | 5 |
| Myritol 318 | -- | - | 3,5 | 3,5 | 3,5 |
| Safloröl raffiniert | - | - | 2 | 2 | 2 |
| 3,3,5-Trimethylcyclohexylsalicylat | 7 | 7 | - | - | - |
| Octocrylene | 10 | 10 | 2 | 2 | 2 |
| Butylmethoxydibenzoylmethan | 4 | 4 | 3 | 3 | 3 |
| Parsol SLX | - | - | 2 | 2 | 2 |
| Neo Heliopan Hydro | 2 | 2 | 2 | 2 | 2 |
| Neo Heliopan AP | 1 | 1 | 1 | 1 | 1 |
| L-Arginin | 1 | 1 | - | 1 | 1 |
| Natriumhydroxid Perlen | 0,24 | 0,24 | 0,28 | 0,46 | 0,34 |
| AMP-ULTRA PC 2000 | - | - | 1 | - | - |
| Cosmedia Silc | 2 | 2 | 2 | 2 | 2 |
| Carbopol ETD 2020 | 0,2 | 0,2 | 0,22 | 0,22 | 0,22 |
| Xanthan | 0,2 | 0,2 | - | - | - |
| Aristoflex AVC | 0,6 | 0,6 | 0,6 | 0,6 | 0,6 |
| Cosmedia SP | - | - | 0,5 | 0,5 | 0,5 |
| Olivem 1000 | - | - | - | - | 5 |
| Tetranatrium-EDTA (Pulver) | 0,1 | 0,1 | 0,1 | 0,1 | 0,1 |
| Ethylparaben | 0,2 | 0,2 | 0,2 | 0,2 | 0,2 |
| Methylparaben | 0,2 | 0,2 | 0,2 | 0,2 | 0,2 |
| Phenoxyethanol | 0,4 | 0,4 | 0,4 | 0,4 | 0,4 |
| RonaCare AP | 0,05 | 0,05 | 0,05 | 0,05 | 0,05 |
| Vitamin E Acetat | 0,5 | 0,5 | 0,5 | 0,5 | 0,5 |
| Controx KS C | 0,05 | 0,05 | 0,05 | 0,05 | 0,05 |

(fortgesetzt)

| | Creme A (erfindungsgemäß | Creme B (V) | Creme C (V) | Creme D (V) | Creme E (V) |
|---|---|---|---|---|---|
| Hexandiol-1,6 | 4 | 4 | 6 | 6 | 6 |
| Glycerin | 5 | 5 | 3,9 | 3,9 | 3,9 |
| Parfum | 0,4 | 0,25 | 0,3 | 0,3 | 0,3 |
| Wasser vollentsalzt | ad 100 | ad 100 | ad 100 | ad 100 | ad 100 |

Die erfindungsgemäße Creme A wurde im Vergleich zur nicht erfindungsgemäßen Creme B mittels eines geschulten Testpanels im Rahmen einer sensorischen Produktbewertung evaluiert.

Die Beurteilungsparameter finden sich in der unten stehenden Grafik wieder. Der Begriff "Wachsigkeit" umschreibt ein stumpfes Hautgefühl bzw. ein "Bremsen" beim Auftragen der Creme auf die Haut. Eine solche Sensorik ist nicht erwünscht. Als Gegenteil der "Wachsigkeit" dient der Parameter "Glätte". Eine glatte Sensorik ist erwünscht. "Einziehen I" bezieht sich auf das Rückstandsverhalten der Creme auf der Haut beim Auftragen. "Einziehen II" bezieht sich auf das Rückstandsverhalten der Creme auf der Haut eine Minute nach dem Auftragen.

Die erfindungsgemäße Creme A wurde gegenüber der nicht erfindungsgemäßen Creme B als weniger wachsig, weniger klebrig, glatter und leichter verteilbar bewertet.

**Sensorisches Profil von Creme A Code 10/15**

**vs Creme B Code 10/02 (=0-Linie), Median**

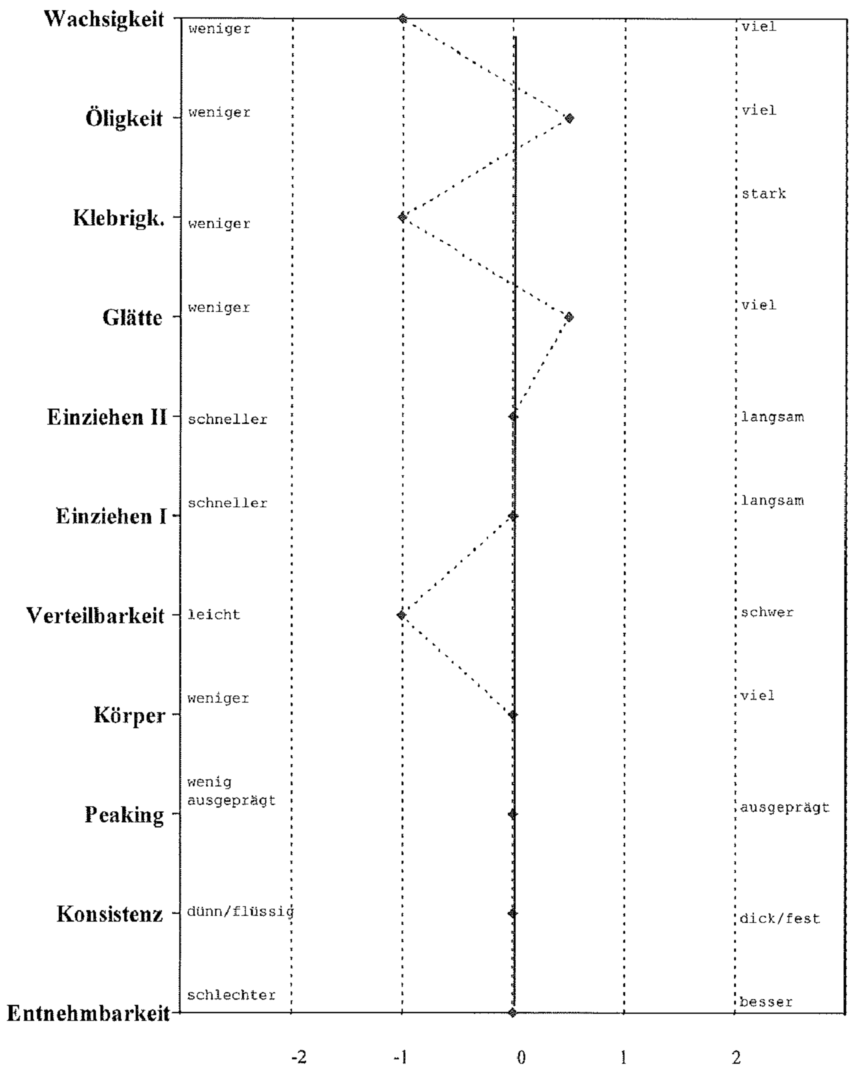


Im Lagertest zeigten sich nur die Cremes A und C als lagerstabil. Creme C war allerdings nicht photostabil. Die Cremes B, D und E waren nicht lagerstabil.
Creme A wies einen *in vivo* SPF von 63,6 und einen *in vitro* UVA-SPF von 20,4 auf.
Creme B wies hingegen nur einen *in vivo* SPF von 40,6 und einen *in vitro* UVA-SPF von 15,1 auf. Für die Cremes C, D und E bemängelte ein Testpanel aus 10 Anwenderinnen, dass die Creme in die Augen migrierte und ein Jucken oder Tränen der Augen hervorrief.

Die Photostabilität wurde in vitro mit dem Gerät UV-Laser 2000 S gemessen.
Die Lagerstabilität wurde bei Temperaturen von 0°C, 40°C, 50°C und 60°C ermittelt.
Die Creme sollte möglichst lange Temperaturstabil sein, 60°C wurde über 4 Wochen beurteilt, die anderen Temperaturmuster lagerten 24 Wochen.

Für eine Markt-akzeptable Lagerstabilität sollte zumindest das bei 40°C gelagerte Muster nach 24 Wochen noch keine Phasentrennung der Emulsionsphasen zeigen.

## Patentansprüche

1. Kosmetische Sonnenschutz-Zusammensetzung in Form einer Öl-in-Wasser-Emulsion, enthaltend die Komponenten a) bis g),

a) mindestens ein Salz eines $C_{12-20}$-Alkylphosphats, insbesondere ein Salz von Cetylphosphat, als anionischen Öl-in-Wasser-Emulgator, in einer Gesamtmenge von 0,5 - 2,0 Gew.-%,
b) mindestens ein $C_{14-20}$-Mono- oder Diacylglycerid, bevorzugt mindestens ein $C_{16-18}$-Mono- oder Diacylglycerid, besonders bevorzugt ausgewählt aus gehärteten Palmölglyceriden, in einer Gesamtmenge von 0,3 - 1,7 Gew.-%,
c) 0,3 - 1 Gew.-% Behenylalkohol,
d) lineare primäre Alkanole mit 12 bis 21 oder mehr als 22 Kohlenstoffatomen in einer Gesamtmenge von 0,03 bis 0,4 Gew.-%,
e) in einer Gesamtmenge von 0,7 - 2 Gew.-% n-Hexadecyl-n-nonanoat, n-Octadecyl-n-nonanoat oder eine Mischung hiervon,
f) 2 - 6 Gew.-% 2-Ethylhexyl-3,5,5-trimethylhexanoat,
g) mindestens eine in der Ölphase oder der Wasserphase gelöste organische UV-Filtersubstanz, darunter 2-Cyano-3,3-phenylzimtsäure-2-ethylhexylester, 4-(tert.-Butyl)-4'-methoxydibenzoylmethan und mindestens eine in der Wasserphase gelöste organische UV-Filtersubstanz, die ausgewählt ist aus Phenylen-1,4-bis-(2-benzimidazyl)-3,3'-5,5'-tetrasulfonsäure, 2-Phenylbenzimidazol-5-sulfonsäure und den Salzen dieser Säuren sowie aus Mischungen der genannten Sulfonsäurederivate, in einer Gesamtmenge von mindestens 15 Gew.-%,

**dadurch gekennzeichnet, dass** die mindestens eine in der Ölphase gelöste organische UV-Filtersubstanz 3,3,5-Trimethylcyclohexylsalicylat (Homosalate) umfasst, wobei sich die Angaben in Gew.-% auf das Gesamtgewicht der Sonnenschutz-Zusammensetzung beziehen.

2. Kosmetische Zusammensetzung gemäß Anspruch 1, **dadurch gekennzeichnet, dass**, bezogen auf das Gesamtgewicht der Sonnenschutz-Zusammensetzung, unter Normalbedingungen feste Ester, die aus linearen Carbonsäuren mit 1 - 8 Kohlenstoffatomen und linearen Alkanolen mit 1 - 18 Kohlenstoffatomen gebildet sind, in einer Gesamtmenge von 0 bis 0,6 Gew.-% enthalten sind.

3. Kosmetische Zusammensetzung gemäß Anspruch 1 oder 2, **dadurch gekennzeichnet, dass** als Komponenten a) und b) ein Gemisch aus Dikaliummonocetylphosphat und Kaliumdicetylphosphat mit gehärteten Palmölglyceriden enthalten ist.

4. Kosmetische Zusammensetzung gemäß Anspruch 1, 2 oder 3, **dadurch gekennzeichnet, dass** 2-Cyano-3,3-phenylzimtsäure-2-ethylhexylester in einer Gesamtmenge von 3 bis 13 Gew.-%, bevorzugt 5 bis 11 Gew.-% und besonders bevorzugt 8 - 10 Gew.-%, 4-(tert.-Butyl)-4'-methoxydibenzoylmethan in einer Gesamtmenge von 0,5 bis 10 Gew.-%, bevorzugt 1 bis 8 Gew.-%, besonders bevorzugt 2 bis 5 Gew.-% und außerordentlich bevorzugt 3 - 4 Gew.-%, die mindestens eine in der Wasserphase gelöste organische UV-Filtersubstanz, die ausgewählt ist aus Phenylen-1,4-bis-(2-benzimidazyl)-3,3'-5,5'-tetrasulfonsäure, 2-Phenylbenzimidazol-5-sulfonsäure und den Salzen dieser Säuren sowie aus Mischungen hiervon, in einer Gesamtmenge von 0,5 - 15 Gew.-%, bevorzugt 1 - 10 Gew.-%, besonders bevorzugt 2 - 5 Gew.-% und außerordentlich bevorzugt 3 - 4 Gew.-%, jeweils bezogen auf den Gehalt dieser Substanzen in ihrer Säureform,
jeweils bezogen auf das Gewicht der gesamten Zusammensetzung, enthalten sind.

5. Kosmetische Zusammensetzung gemäß Anspruch 1, 2, 3 oder 4, **dadurch gekennzeichnet, dass** 3,3,5-Trimethylcyclohexylsalicylat in einer Gesamtmenge von 0,5 - 10 Gew.-%, bevorzugt 2 - 9 Gew.-%, besonders bevorzugt 4 - 8 Gew.-%, außerordentlich bevorzugt 5,5 - 7 Gew.-%, bezogen auf das Gewicht der gesamten Zusammensetzung, enthalten ist.

6. Kosmetische Zusammensetzung gemäß Anspruch 1, 2, 3, 4 oder 5, **dadurch gekennzeichnet, dass** sie frei von 2-Ethylhexylsalicylat, frei von 4-Isopropylbenzylsalicylat, frei von Polysilicone-15 und frei von öllöslichen organischen UV-Filtersubstanzen auf Basis von s-Triazinderivaten, die das nachfolgende Strukturmotiv TRIAZIN-GRUND aufweisen, ist.

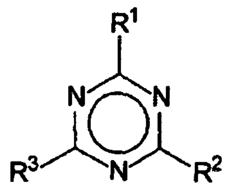

TRIAZIN-GRUND

7. Kosmetische Zusammensetzung gemäß Anspruch 1, 2, 3, 4, 5 oder 6, **dadurch gekennzeichnet, dass**, bezogen auf das Gesamtgewicht der Zusammensetzung, 0,1 bis 5 Gew.-% unter Normalbedingungen fester, ungelöster Partikel mit einem zahlenmittleren Durchmesser von mindestens 350 nm, die in der Zusammensetzung unlöslich sind, enthalten sind.

8. Kosmetische Zusammensetzung gemäß Anspruch 1, 2, 3, 4, 5, 6 oder 7, **dadurch gekennzeichnet, dass** eine Mischung aus Xanthan-Gum, mindestens einem vernetzten Polyacrylat und mindestens einem vernetzten Acryloyldimethyltaurat-Copolymer enthalten ist, bevorzugt in einer Gesamtmenge von 0,5 - 1,5 Gew.-%, besonders bevorzugt 0,7 - 1,3 Gew.-% und außerordentlich bevorzugt 1 - 1,2 Gew.-%, jeweils bezogen auf das Gesamtgewicht der Zusammensetzung.

9. Kosmetische Zusammensetzung gemäß Anspruch 1, 2, 3, 4, 5, 6, 7 oder 8, **dadurch gekennzeichnet, dass**, jeweils bezogen auf das Gesamtgewicht der Zusammensetzung, eine Mischung aus Dikaliummonocetylphosphat und Kaliumdicetylphosphat in einer Gesamtmenge von 0,8 -1,8 Gew.-%, bevorzugt 1,2 - 1,5 Gew.-%, weiterhin in einer Gesamtmenge von 0,6 - 1,4 Gew.-%, bevorzugt 0,9 - 1,1 Gew.-%, mindestens eine Substanz, ausgewählt aus Monomyristoylglycerid, Monopalmitoylglycerid, Monostearoylglycerid, Monoarachinoylglycerid, Dimyristoylglycerid, Dipalmitoylglycerid, Distearoylglycerid und Diarachinoylglycerid sowie aus Mischungen hiervon, enthalten ist.

10. Kosmetische Zusammensetzung gemäß Anspruch 1, 2, 3, 4, 5, 6, 7, 8 oder 9, **dadurch gekennzeichnet, dass**, jeweils bezogen auf das Gesamtgewicht der Zusammensetzung, eine Mischung aus Dikaliummonocetylphosphat und Kaliumdicetylphosphat in einer Gesamtmenge von 0,8 -1,8 Gew.-%, bevorzugt 1,2 - 1,5 Gew.-%, weiterhin in einer Gesamtmenge von 0,6 - 1,4 Gew.-%, bevorzugt 0,9 - 1,1 Gew.-%, mindestens eine Substanz, ausgewählt aus gehärteten Palmölglyceriden, enthalten ist.

11. Kosmetische Zusammensetzung gemäß Anspruch 1, 2, 3, 4, 5, 6, 7, 8, 9 oder 10, **dadurch gekennzeichnet, dass** 2,5 - 5 Gew.-%, bevorzugt 3 - 4 Gew.-% 2-Ethylhexyl-3,5,5-trimethylhexanoat enthalten sind, jeweils bezogen auf das Gesamtgewicht der Zusammensetzung.

12. Kosmetische Zusammensetzung gemäß Anspruch 1, 2, 3, 4, 5, 6, 7, 8, 9, 10 oder 11, **gekennzeichnet durch** einen pH-Wert bei 20°C im Bereich von 5,7 - 7,3, bevorzugt im Bereich von 6,0 bis 6,7, besonders bevorzugt im Bereich von 6,2 bis 6,4.

13. Kosmetische Zusammensetzung gemäß Anspruch 1, 2, 3, 4, 5, 6, 7, 8, 9, 10, 11 oder 12, **dadurch gekennzeichnet, dass** ein Gemisch aus Dikaliummonocetylphosphat und Kaliumdicetylphosphat mit gehärteten Palmölglyceriden in einer Gesamtmenge von 0,5 - 4 Gew.-%, besonders bevorzugt 1 - 3,7 Gew.-%, insbesondere 2 - 3 Gew.-% und außerordentlich bevorzugt 2 - 2,5 Gew.-%, weiterhin 2-Cyano-3,3-phenylzimtsäure-2-ethylhexylester in einer Gesamtmenge von 0,5 - 15 Gew.-%, besonders bevorzugt 3 - 13 Gew.-%, insbesondere 5 - 11 Gew.-% und außerordentlich bevorzugt 8 - 10 Gew.-%, 4-tert-Butyl-4'-methoxydibenzoylmethan in einer Gesamtmenge von 0,5 - 10 Gew.-%, bevorzugt 1 - 8 Gew.-%, besonders bevorzugt 2 - 5 Gew.-% und außerordentlich bevorzugt 3 - 4 Gew.-%, 3,3,5-Trimethylcyclohexylsalicylat in einer Gesamtmenge von 0,5 - 10 Gew.-%, bevorzugt 2 - 9 Gew.-%, besonders bevorzugt 4 - 8 Gew.-%, außerordentlich bevorzugt 5,5 - 7 Gew.-%, das Argininsalz der 2-Phenylbenzimidazol-5-sulfonsäure in einer Gesamtmenge von 0,5 - 8 Gew.-%, bevorzugt 1 - 6 Gew.-%, besonders bevorzugt 1,5 - 4 Gew.-% und außerordentlich bevorzugt 2 - 3 Gew.-%, und Dinatriumphenylbenzimidazoltetrasulfonat in einer Gesamtmenge von 0,1 - 10 Gew.-%, bevorzugt 0,5 - 8 Gew.-%, besonders bevorzugt 0,8 - 5 Gew.-% und außerordentlich bevorzugt 0,9 - 2 Gew.-%, jeweils bezogen auf das Gesamtgewicht der erfindungsgemäßen Zusammensetzung, enthalten sind und die Zusammensetzung einen pH-Wert bei 20°C von 5,7 - 7,3, bevorzugt 6,0 - 6,7, besonders bevorzugt 6,2 bis 6,4, aufweist.

14. Kosmetische Verwendung einer Zusammensetzung gemäß einem der Ansprüche 1, 2, 3, 4, 5, 6, 7, 8, 9, 10, 11, 12 oder 13 zur Verzögerung der Lichtalterung der Haut und/oder zur kontrollierten Bräunung der Haut.

**Claims**

1. A cosmetic sunscreen composition in the form of an oil-in-water emulsion, containing the components a) to g),

a) at least one salt of a $C_{12-20}$ alkyl phosphate, in particular a salt of cetyl phosphate, as an anionic oil-in-water emulsifier, in a total amount of from 0.5 to 2.0 wt.%,

b) at least one $C_{14-20}$ monoacylglyceride or diacylglyceride, preferably at least one $C_{16-18}$ monoacylglyceride or diacylglyceride, particularly preferably selected from hydrogenated palm glycerides, in a total amount of from 0.3 to 1.7 wt.%,
c) 0.3 to 1 wt.% behenyl alcohol,
d) linear primary alkanols having 12 to 21 or more than 22 carbon atoms in a total amount of from 0.03 to 0.4 wt.%,
e) in a total amount of from 0.7 to 2 wt.%, n-hexadecyl-n-nonanoate, n-octadecyl-n-nonanoate or a mixture thereof,
f) 2 to 6 wt.% 2-ethylhexyl-3,5,5-trimethylhexanoate,
g) at least one organic UV filter substance dissolved in the oil phase or the water phase, including 2-cyano-3,3-phenyl cinnamic acid-2-ethylhexyl ester, 4-(tert-butyl)-4'-methoxydibenzoylmethane and at least one organic UV filter substance dissolved in the water phase, which substance is selected from phenylene-1,4-bis-(2-benzimidazole)-3,3'-5,5'-tetrasulfonic acid, 2- phenylbenzimidazole-5-sulfonic acid and the salts of said acids, and from mixtures of the mentioned sulfonic acid derivatives, in a total amount of at least 15 wt.%,

**characterized in that** the at least one organic UV filter substance dissolved in the oil phase comprises 3,3,5-trimethylcyclohexyl salicylate (homosalate), the amounts in wt.% relating to the total weight of the sunscreen composition.

2. The cosmetic composition according to claim 1, **characterized in that**, based on the total weight of the sunscreen composition, under normal conditions, solid esters, which are formed of linear carboxylic acids having 1 to 8 carbon atoms and linear alkanols having 1 to 18 carbon atoms, are contained in a total amount of from 0 to 0.6 wt.%.

3. The cosmetic composition according to claim 1 or 2, **characterized in that**, as components a) and b), a mixture of dipotassium monocetyl phosphate and potassium dicetyl phosphate with hydrogenated palm glycerides is contained.

4. The cosmetic composition according to claim 1, 2 or 3, **characterized in that** 2-cyano-3,3-phenyl cinnamic acid-2-ethylhexyl ester is contained in a total amount of from 3 to 13 wt.%, preferably 5 to 11 wt.% and particularly preferably 8 to 10 wt.%, 4-(tert-butyl)-4'-methoxydibenzoylmethane is contained in a total amount of from 0.5 to 10 wt.%, preferably 1 to 8 wt.%, particularly preferably 2 to 5 wt.% and extremely preferably 3 to 4 wt.%, the at least one organic UV filter substance dissolved in the water phase, which is selected from phenylene-1,4-bis-(2-benzimidazole)-3,3'-5,5'-tetrasulfonic acid, 2-phenylbenzimidazole-5-sulfonic acid and the salts of said acids, and from mixtures thereof, is contained in a total amount of from 0.5 to 15 wt.%, preferably 1 to 10 wt.%, particularly preferably 2 to 5 wt.% and extremely preferably 3 to 4 wt.%, in each case based on the content of these substances in the acid form thereof, in each case based on the weight of the overall composition.

5. The cosmetic composition according to claim 1, 2, 3 or 4, **characterized in that** 3,3,5-trimethylcyclohexyl salicylate is contained in a total amount of from 0.5 to 10 wt.%, preferably 2 to 9 wt.%, particularly preferably 4 to 8 wt.%, extremely preferably 5.5 to 7 wt.%, based on the weight of the total composition.

6. The cosmetic composition according to claim 1, 2, 3, 4 or 5, **characterized in that** said composition is free of 2-ethylhexyl salicylate, free of 4-isopropylbenzyl salicylate, free of polysilicone-15 and free of oil-soluble organic UV filter substances based on s-triazine derivatives, which have the following structural motif TRIAZINE BASE.

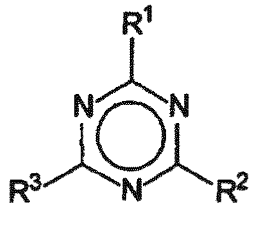

TRIAZINE BASE

7. The cosmetic composition according to claim 1, 2, 3, 4, 5 or 6, **characterized in that**, based on the total weight of the composition, 0.1 to 5 wt.% under normal conditions of solid, undissolved particles which have a number average diameter of at least 350 nm and are insoluble in the composition, are contained.

8. The cosmetic composition according to claim 1, 2, 3, 4, 5, 6 or 7, **characterized in that** a mixture of xanthan gum, at least one crosslinked polyacrylate and at least one crosslinked acryloyl dimethyltaurate copolymer is contained,

preferably in a total amount of from 0.5 to 1.5 wt.%, particularly preferably 0.7 to 1.3 wt.% and extremely preferably 1 to 1.2 wt.%, in each case based on the total weight of the composition.

**9.** The cosmetic composition according to claim 1, 2, 3, 4, 5, 6, 7 or 8, **characterized in that**, in each case based on the total weight of the composition, a mixture of dipotassium monocetyl phosphate and potassium dicetyl phosphate is contained in a total amount of from 0.8 to 1.8 wt.%, preferably 1.2 to 1.5 wt.%, additionally, in a total amount of from 0.6 to 1.4 wt.%, preferably 0.9 to 1.1 wt.%, at least one substance, selected from monomyristoyl glyceride, monopalmitoyl glyceride, monostearoyl glyceride, monoarachinoyl glyceride, dimyristoyl glyceride, dipalmitoyl glyceride, distearoyl glyceride and diarachinoyl glyceride, and from mixtures thereof, is contained.

**10.** The cosmetic composition according to claim 1, 2, 3, 4, 5, 6, 7, 8 or 9, **characterized in that**, in each case based on the total weight of the composition, a mixture of dipotassium monocetyl phosphate and potassium dicetyl phosphate is contained in a total amount of from 0.8 to 1.8 wt.%, preferably 1.2 to 1.5 wt.%, additionally, in a total amount of from 0.6 to 1.4 wt.%, preferably 0.9 to 1.1 wt.%, at least one substance, selected from hydrogenated palm glycerides, is contained.

**11.** The cosmetic composition according to claim 1, 2, 3, 4, 5, 6, 7, 8, 9 or 10, **characterized in that** 2.5 to 5 wt.%, preferably 3 to 4 wt.%, of 2-ethylhexyl-3,5,5-trimethylhexanoate is contained, in each case based on the total weight of the composition.

**12.** The cosmetic composition according to claim 1, 2, 3, 4, 5, 6, 7, 8, 9, 10 or 11, **characterized by** a pH at 20 °C in the range of from 5.7 to 7.3, preferably in the range of from 6.0 to 6.7, particularly preferably in the range of from 6.2 to 6.4.

**13.** The cosmetic composition according to claim 1, 2, 3, 4, 5, 6, 7, 8, 9, 10, 11 or 12, **characterized in that** a mixture of dipotassium monocetyl phosphate and potassium dicetyl phosphate with hydrogenated palm glycerides is contained in a total amount of from 0.5 to 4 wt.%, particularly preferably 1 to 3.7 wt.%, in particular 2 to 3 wt.% and extremely preferably 2 to 2.5 wt.%, additionally 2-cyano-3,3-phenyl cinnamic acid-2-ethylhexyl ester is contained in a total amount of from 0.5 to 15 wt.%, particularly preferably 3 to 13 wt.%, in particular 5 to 11 wt.% and extremely preferably 8 to 10 wt.%, 4-tert-butyl-4'-methoxydibenzoylmethane is contained in a total amount of from 0.5 to 10 wt.%, preferably 1 to 8 wt.%, particularly preferably 2 to 5 wt.% and extremely preferably 3 to 4 wt.%, 3,3,5-trimethylcyclohexyl salicylate is contained in a total amount of from 0.5 to 10 wt.%, preferably 2 to 9 wt.%, particularly preferably 4 to 8 wt.%, extremely preferably 5.5 to 7 wt.%, the arginine salt of 2-phenylbenzimidazole-5-sulfonic acid is contained in a total amount of from 0.5 to 8 wt.%, preferably 1 to 6 wt.%, particularly preferably 1.5 to 4 wt.% and extremely preferably 2 to 3 wt.%, and disodium phenyl benzimidazole tetrasulfonate is contained in a total amount of from 0.1 to 10 wt.%, preferably 0.5 to 8 wt.%, particularly preferably 0.8 to 5 wt.% and extremely preferably 0.9 to 2 wt.%, in each case based on the total weight of the composition according to the invention, and the composition has a pH at 20 °C of 5.7 to 7.3, preferably 6.0 to 6.7, particularly preferably 6.2 to 6.4.

**14.** The cosmetic use of a composition according to one of claims 1, 2, 3, 4, 5, 6, 7, 8, 9, 10, 11, 12 or 13 for delaying light ageing of the skin and/or for controlled tanning of the skin.

## Revendications

**1.** Composition cosmétique de protection solaire sous la forme d'une émulsion huile-dans-eau, contenant les composants a) à g),

a) au moins un sel d'un phosphate d'alkyle en $C_{12-20}$, en particulier un sel de phosphate de cétyle, en tant qu'émulsifiant huile-dans-eau anionique, dans une quantité totale allant de 0,5 - 2,0 % en poids,
b) au moins un mono- ou diacylglycéride en $C_{14-20}$, de manière préférée au moins un mono- ou diacylglycéride en $C_{16-18}$, de manière particulièrement préférée choisi parmi les glycérides d'huile de palme durcis, dans une quantité totale allant de 0,3 - 1,7 % en poids,
c) 0,3 - 1 % en poids d'alcool béhénylique,
d) des alcanols primaires linéaires ayant de 12 à 21 ou plus de 22 atomes de carbone dans une quantité totale allant de 0,03 à 0,4 % en poids,
e) dans une quantité totale allant de 0,7 à 2 % en poids, des n-hexadécyl-n-nonanoate, des n-octadécyl-n-nonanoate ou un mélange de ces derniers,

f) 2-6 % en poids de 2-éthylhexyl-3,5,5-triméthylhexanoate,
g) au moins une substance organique de filtre UV dissoute dans la phase huileuse ou dans la phase aqueuse, comprenant l'ester de 2-cyano-3,3-phénylcinnamate de 2-éthylhexyle, le 4-(tert-butyl)-4'-méthoxydibenzoylméthane et au moins une substance organique de filtre UV dissoute dans la phase aqueuse, qui est choisie parmi l'acide phénylène-1,4-bis-(2-benzimidazyl)-3,3'-5,5'-tétrasulfonique, l'acide 2-phénylbenzimidazole-5-sulfonique et les sels de ces acides ainsi que parmi les mélanges desdits dérivés d'acide sulfonique, dans une quantité totale d'au moins 15 % en poids,

**caractérisé en ce que** l'au moins une substance organique de filtre UV dissoute dans la phase huileuse comprend du 3,3,5-triméthylcyclohexylsalicylate (homosalates), les données étant exprimées en % du poids total de la composition de protection solaire.

**2.** Composition cosmétique selon la revendication 1, **caractérisée en ce que**, rapporté au poids total de la composition de protection solaire, elle contient dans des conditions normales des esters solides formés d'acides carboxyliques linéaires ayant 1 à 8 atomes de carbone et d'alcanols linéaires ayant 1 à 18 atomes de carbone, dans une quantité totale allant de 0 à 0,6 % en poids.

**3.** Composition cosmétique selon la revendication 1 ou 2, **caractérisée en ce qu'**elle contient, en tant que composants a) et b), un mélange de monocétylphosphate dipotassique et de dicétylphosphate de potassium avec des glycérides durcis d'huile de palme.

**4.** Composition cosmétique selon la revendication 1, 2 ou 3, **caractérisée en ce qu'**elle contient de l'ester 2-éthylhexyle de l'acide 2-cyano-3,3-phénylcinnamique dans une quantité totale allant de 3 à 13 % en poids, de manière préférée de 5 à 11 % en poids et de manière particulièrement préférée de 8 - 10 % en poids, du 4-(tert.-butyl)-4'-méthoxydibenzoylméthane dans une quantité totale allant de 0,5 à 10 % en poids, de manière préférée de 1 à 8 % en poids, de manière particulièrement préférée de 2 à 5 % en poids et de manière préférée entre toutes de 3 - 4 % en poids, l'au moins une substance organique de filtre UV dissoute dans la phase aqueuse, qui est choisie parmi l'acide phénylène-1,4-bis (2-benzimidazyl)-3,3'-5,5'-tétrasulfonique, l'acide 2-phénylbenzimidazole-5-sulfonique et les sels de ces acides ainsi que parmi les mélanges de ces derniers, dans une quantité totale allant de 0,5 - 15 % en poids, de manière préférée de 1 - 10 % en poids, de manière particulièrement préférée de 2 - 5 % en poids et de manière préférée entre toutes de 3 - 4 % en poids, respectivement rapporté à la teneur de ces substances dans leur forme acide, respectivement rapporté au poids de l'ensemble de la composition.

**5.** Composition cosmétique selon la revendication 1, 2, 3 ou 4, **caractérisée en ce qu'**elle contient du 3,3,5-triméthylcyclohexylsalicylate dans une quantité totale allant de 0,5 - 10 % en poids, de manière préférée de 2 - 9 % en poids, de manière particulièrement préférée de 4 - 8 % en poids, de manière préférée entre toutes de 5,5 - 7 % en poids, rapporté au poids de l'ensemble de la composition.

**6.** Composition cosmétique selon la revendication 1, 2, 3, 4 ou 5, **caractérisée en ce qu'**elle ne contient aucune trace de salicylate de 2-éthylhexyle, de 4-isopropylbenzylsalicylate, de polysilicone-15 et de substances organiques de filtre UV solubles dans l'huile à base de dérivés s-triazine qui présentent le motif structural suivant BASE TRIAZINE.

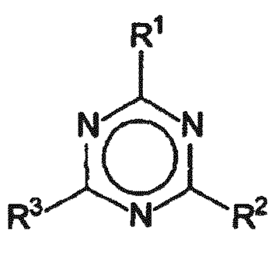

BASE TRIAZINE

**7.** Composition cosmétique selon la revendication 1, 2, 3, 4, 5 ou 6, **caractérisée en ce qu'**elle contient, rapporté au poids total de la composition, de 0,1 à 5 % en poids de particules solides non dissoutes en conditions normales ayant un diamètre moyen en nombre d'au moins 350 nm, qui sont insolubles dans la composition.

**8.** Composition cosmétique selon la revendication 1, 2, 3, 4, 5, 6 ou 7, **caractérisée en ce qu'**elle contient un mélange de gomme de xanthane, d'au moins un polyacrylate réticulé et d'au moins un copolymère acryloyldiméthyltaurate réticulé, de manière préférée dans une quantité totale allant de 0,5 - 1,5 % en poids, de manière particulièrement

préférée de 0,7 - 1,3 % en poids, et de manière préférée entre toutes de 1 - 1,2 % en poids, respectivement rapporté au poids total de la composition.

9. Composition cosmétique selon la revendication 1, 2, 3, 4, 5, 6, 7 ou 8, **caractérisée en ce qu'**elle contient, respectivement rapporté au poids total de la composition, un mélange de monocétylphosphate dipotassique et de dicétylphosphate de potassium dans une quantité totale allant de 0,8 - 1,8 % en poids, de manière préférée de 1,2 - 1,5 % en poids, en outre dans une quantité totale allant de 0,6 - 1,4 % en poids, de manière préférée de 0,9 - 1,1 % en poids, au moins une substance choisie parmi le monomyristoylglycéride, le monopalmitoylglycéride, le monostéaroylglycéride, le monoarachinoylglycéride, le dimyristoylglycéride, le dipalmitoylglycéride, le distéaroylglycéride et le diarachinoylglycéride, ainsi que parmi les mélanges de ces derniers.

10. Composition cosmétique selon la revendication 1, 2, 3, 4, 5, 6, 7, 8 ou 9, **caractérisée en ce qu'**elle contient, respectivement rapporté au poids total de la composition, un mélange de monocétylphosphate dipotassique et de dicétylphosphate de potassium dans une quantité totale allant de 0,8 - 1,8 % en poids, de manière préférée de 1,2 - 1,5 % en poids, en outre dans une quantité totale allant de 0,6 - 1,4 % en poids, de manière préférée de 0,9 - 1,1 % en poids, au moins une substance choisie parmi les glycérides durcis d'huile de palme.

11. Composition cosmétique selon la revendication 1, 2, 3, 4, 5, 6, 7, 8, 9 ou 10, **caractérisée en ce qu'**elle contient de 2,5 - 5 % en poids, de manière préférée de 3 - 4 % en poids de 2-éthylhexyle-3,5,5-triméthylhexanoate, respectivement rapporté au poids total de la composition.

12. Composition cosmétique selon la revendication 1, 2, 3, 4, 5, 6, 7, 8, 9, 10 ou 11, **caractérisée par** une valeur pH à 20 °C comprise dans une plage allant de 5,7 - 7,3, de manière préférée dans une plage allant de 6,0 à 6,7, de manière particulièrement préférée dans une plage allant de 6,2 à 6,4.

13. Composition cosmétique selon la revendication 1, 2, 3, 4, 5, 6, 7, 8, 9, 10, 11 ou 12, **caractérisée en ce qu'**elle contient un mélange de monocétylphosphate dipotassique et de dicétylphosphate de potassium avec des glycérides durcis d'huile de palme dans une quantité totale allant de 0,5 - 4 % en poids, de manière particulièrement préférée 1 - 3,7 % en poids, en particulier 2 - 3 % en poids et de manière préférée entre toutes 2 - 2,5 % en poids, en outre de l'ester de 2-cyano-3,3-phénylcinnamate de 2-éthylhexyle dans une quantité totale allant de 0,5 - 15 % en poids, de manière particulièrement préférée de 3 - 13 % en poids, en particulier de 5 - 11 % en poids et de manière préférée entre toutes de 8 - 10 % en poids, du 4-tert-butyle-4'-méthoxydibenzoylméthane dans une quantité totale allant de 0,5 - 10 % en poids, de manière préférée de 1 - 8 % en poids, de manière particulièrement préférée de 2 - 5 % en poids et de manière préférée entre toutes de 3 - 4 % en poids, du 3,3,5-triméthylcyclohexyl-salicylate dans une quantité totale allant de 0,5 - 10 % en poids, de manière préférée de 2 - 9 % en poids, de manière particulièrement préférée de 4 - 8 % en poids, de manière préférée entre toutes de 5,5 - 7 % en poids, le sel d'arginine de l'acide 2-phénylbenzimidazole-5-sulfonique dans une quantité totale allant de 0,5 - 8 % en poids, de manière préférée de 1 - 6 % en poids, de manière particulièrement préférée de 1,5 - 4 % en poids et de manière préférée entre toutes de 2 - 3 % en poids, et du phénylbenzimidazole tétrasulfonate disodique dans une quantité totale allant de 0,1 - 10 % en poids, de manière préférée de 0,5 - 8 % en poids, de manière particulièrement préférée de 0,8 - 5 % en poids et de manière préférée entre toutes de 0,9 - 2 % en poids, respectivement rapporté au poids total de la composition selon l'invention, et la composition présente une valeur pH à 20 °C allant de 5,7 - 7,3, de manière préférée de 6,0 - 6,7, de manière particulièrement préférée de 6,2 - 6,4.

14. Utilisation cosmétique d'une composition selon l'une des revendications 1, 2, 3, 4, 5, 6, 7, 8, 9, 10, 11, 12 ou 13 pour retarder le photo-vieillissement de la peau et/ou pour un bronzage contrôlé de la peau.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- EP 2201927 A2
- EP 709080 A2
- EP 775698 A
- EP 878469 A
- EP 1027881 A

### In der Beschreibung aufgeführte Nicht-Patentliteratur

- *CHEMICAL ABSTRACTS,* 207574-74-1
- *CHEMICAL ABSTRACTS,* 288254-16-0
- *CHEMICAL ABSTRACTS,* 025973-551
- *CHEMICAL ABSTRACTS,* 003147-75-9
- *CHEMICAL ABSTRACTS,* 2440-22-4
- *CHEMICAL ABSTRACTS,* 155633-54-8